(19) Europäisches Patentamt
European Patent Office
Office européen des brevets

(11) **EP 1 730 219 B1**

(12) **EUROPEAN PATENT SPECIFICATION**

(45) Date of publication and mention
of the grant of the patent:
**03.02.2016 Bulletin 2016/05**

(51) Int Cl.:
***C08J 3/12*** *(2006.01)*      ***C08J 3/24*** *(2006.01)*
***A61L 15/60*** *(2006.01)*      ***C08L 101/14*** *(2006.01)*

(21) Application number: **05721709.3**

(22) Date of filing: **29.03.2005**

(86) International application number:
**PCT/JP2005/006570**

(87) International publication number:
**WO 2005/092956 (06.10.2005 Gazette 2005/40)**

(54) **PARTICULATE WATER ABSORBING AGENT WITH WATER-ABSORBING RESIN AS MAIN COMPONENT**

TEILCHENFÖRMIGES WASSERABSORPTIONSMITTEL MIT WASSERABSORBIERENDEM HARZ ALS HAUPTKOMPONENTE

AGENT PARTICULAIRE D'ABSORPTION D'EAU AVEC UNE RESINE D'ABSORPTION D'EAU EN TANT QUE COMPOSANT PRINCIPAL

(84) Designated Contracting States:
**BE DE**

(30) Priority: **29.03.2004 JP 2004096083**
**20.07.2004 JP 2004211856**

(43) Date of publication of application:
**13.12.2006 Bulletin 2006/50**

(73) Proprietor: **Nippon Shokubai Co.,Ltd.**
**Osaka-shi, Osaka 541-0043 (JP)**

(72) Inventors:
• **WADA, Katsuyuki**
**Hyogo 6700081 (JP)**
• **KIMURA, Kazuki;**
**Himeji-shi, Hyogo 6711242; (JP)**
• **UEDA, Hiroko**
**Himeji-shi, Hyogo 6711145 (JP)**
• **KANTO, Teruyuki**
**Himeji-shi, Hyogo 6711235 (JP)**
• **FUJIMARU, Hirotama**
**Osaka 5960807 (JP)**

(74) Representative: **Mai, Dörr, Besier**
**Patentanwälte**
**Steuerberater/Wirtschaftsprüfer**
**Kreuzberger Ring 64**
**65205 Wiesbaden (DE)**

(56) References cited:
**US-B2- 6 599 989**

**Description**

BACKGROUND OF THE INVENTION

Field of the Invention:

The present invention relates to a particulate water absorbing agent including a water-absorbing resin as a main component. In particular, it relates to a particulate water absorbing agent fulfilling superior absorption ability conventionally not obtained in practical applications as absorbing articles such as a diaper.

Description of Related Art:

At present, as component materials in sanitary goods such as a paper diaper, a sanitary napkin, an incontinence pad, and the like, a water-absorbing resin to absorb body fluid and hydrophilic fibers such as pulp are widely used. As the water-absorbing resin, for example, partially neutralized and crosslinked polyacrylic acid, hydrolysates of starch-acrylic acid graft polymer, saponified vinyl acetate-acrylic acid ester copolymers, hydrolysates of acrylonitrile copolymers or acrylamide copolymers or crosslinked polymers thereof, crosslinked polymers of cationic monomers, and the like are used as main raw materials.

The water-absorbing resin has conventionally been required to have superior property such as superior liquid absorption amount or water absorption speed, gel strength and gel permeability in contact with aqueous liquid such as body fluid, along with water suction force to suck water from a substrate containing aqueous liquid. Further, as recent trends, a water-absorbing resin powder with very narrow particle size distribution or a water-absorbing resin with high absorption capacity and low water-extractables has been required and high absorbency against pressure or liquid permeability under pressure has essentially been required. In addition to the above needs, such characteristics of gel has also been required as not to be deteriorated for long term and to retain absorbing performance even in swollen and gelled state by absorption of body fluid or urine.

For example, there are many patent applications on many parameters specifying various properties of these water-absorbing resins or water absorbing agents with a water-absorbing resin as a main component, or on measurement methods thereof (US Reissued 32649, UK2267094B, US5051259, US 5419956, US6087002, EP0629441, EP0707603, EP0712659, EP1029886, US5462972, US5453323, US5797893, US6127454, US6184433, US6297335, US Reissued 37021, US5140076, US6414214B1, US5994440, US6444744, US6194531, EP0940148, EP1153656, EP0605215, US5147343, US5149335, EP0532002, US5601452, US5562646, US5669894, US6150582, WO02/053198, EP0937739).

Water-absorbing resins superior in gel strength, extractables and absorption capacity are proposed in US Reissued 32649. A water-absorbing resin superior in liquid permeability under no pressure, absorption speed and absorption capacity is proposed in UK2267094B. Technology specifying specific particle size distribution is also proposed in US5051259, US 5419956, US6087002 and EP0629441. Further, a water-absorbing resin superior in absorbency against pressure under various loads or many measurement methods therefore are also proposed and water-absorbing resins with superior absorbency against pressure alone or in combination with other property are proposed in EP0707603, EP0712659, EP1029886, US5462972, US5453323, US5797893, US6127454, US6184433, US6297335 and US Reissued 37021.

Water-absorbing resins with little property decrease by impact are proposed in US5140076 and US6414214B1. A water-absorbing resin with specific powdery dust amount is proposed in US5994440, and a water-absorbing resin with less coloring is proposed in US6444744. Water-absorbing resins superior in gel durability in an aqueous L-ascorbic acid solution as index of urine resistance or superior in water absorption ability are proposed in US6194531 and EP0940148. A water-absorbing resin with superior air permeability is proposed in EP1153656. A water-absorbing resin with less residual monomers is proposed in EP0605215.

Further, in US5147343, US5149335, EP0532002,US5601452, US5562646, US5669894, US6150582, WO02/053198, water-absorbing resins with specific property are proposed as suitable to water-absorbing articles such as a diaper having specific property, composition or polymer concentration. Further, a method for surface crosslinking, while pulverizing at least a part of resin particles is proposed in EP0937739.

US-6599989 A1 discloses a waterabsorbent agent prepared from internally crosslinked water-absorbent resin that has been dried and then surface crosslinked. It has a static deterioration absorption capacity under load of 30 g/g or more as determined using a physiological sodium chloride solution containing L-ascorbic acid in concentration of 0.05 % by weight.

While the performance of such agent is quite good the overall performance still can be improved, for instance with respect to avoiding gel blocking or loss by scattering in production of absorbing articles.

SUMMARY OF THE INVENTION

Among water-absorbing resins or water absorbing agents which have been developed based on many properties, as described above, those targeted to or with specifications of these properties have also been produced, however, there was a problem that they have not yet satisfactorily fulfilled performance in practical use such as a paper diaper, and the like, even if these properties are controlled.

Practically sufficient performance has not yet attained even by controlling or designing such properties as water-absorption speed, centrifuge retention capacity, absorbency against pressure, gel strength, durability, extractables and particle size whereas many water-absorbing resins or water absorbing agents have been developed and used. Therefore, it is an object of the present invention to provide a water absorbing agent suitable to practical use in conventional water absorbing agent substrate.

Description of the Preferred Embodiment(s)

After studying to solve the problems, it was found that in a water absorbing agent of the present invention having specific particle size distribution and absorption capacity, components contained in urine gradually destruct crosslinked structure of a water-absorbing resin to make labile to deterioration of the water absorbing agent and such deterioration caused by urine increases extractables and changes water-absorption properties. Conventionally, properties of the water-absorbing resin or the water absorbing agent measured by a physiological saline solution (an aqueous solution of 0.9% by weight of NaCl) or various artificial urine solutions have been proposed, however, urine components in artificial urine are different in each patent and composition of practical urine solution itself is not constant but changes depending on living environment, food practice, age or seasons and furthermore largely changes every time or by physical conditions even in the same person. Because absorption properties of a conventional water-absorbing resin or a water absorbing agent have been evaluated using single kind of specific absorption liquid such as a specific physiological saline solution or artificial urine solution as a urine model, evaluation of the water absorbing agent responsive to change in urine composition could not suitably be carried out and thus it was clarified that a conventional water absorbing agent could not exert sufficient performance in practical use when extractables of the water absorbing agent changed due to change in urine composition.

Therefore, the present invention defined "deterioration caused by urine" as deterioration of a water absorbing agent generated by personal difference in urine, or urine compositional change by seasons or physical conditions of even the same person, and as indexes of degree of deterioration caused by urine, "increased extractables by deterioration" and "increased ratio of extractables by deterioration" were introduced. In the present invention, increased extractables by deterioration and increased ratio of extractables by deterioration are defined by the following formulas. In the present invention, deterioration test liquid means a physiological saline solution containing 0.05% by weight of L-ascorbic acid and a physiological saline solution means an aqueous solution of 0.9% by weight of NaCl, which is used at room temperature (25°C±2°C) unless otherwise specified especially. Measurement methods thereof are as described in Examples shown later.

Increased extractables by deterioration (% by weight) = extractables for one hour in deterioration test liquid (% by weight) – extractables for one hour in a physiological saline solution (% by weight)

Increased ratio of extractables by deterioration = extractables for one hour in deterioration test liquid (% by weight)/extractables for one hour in a physiological saline solution (% by weight)

When extractables increases from the amount before deterioration, in deterioration test liquid, the extractables tends to be more easily exuded from an absorbing substrate, which in turn obstructs diffusion of liquid such as blood and urine into an absorbing substrate and thus lowers absorption properties. In addition, significant increase in extractables suggests destruction of crosslinked structure of the water absorbing agent, making retention difficult of body fluid such as urine absorbed by the water absorbing agent and thus absorption performance is lowered. Such decrease in absorption performance appears as increase in rewet amount in an absorbing substrate or absorbing articles. It is thus preferable for "increased extractables by deterioration" and "increased ratio of extractables by deterioration" to be limited within specific range to inhibits increase in rewet amount.

It is also clarified by detailed study on deterioration due to urine that the deterioration degree tends to depend on surface area of the water absorbing agent and extractables by the deterioration significantly increases in the water absorbing agent with particle size distribution of smaller mass median particle size, for example, the water absorbing agent with mass median particle size D50 not larger than 400 $\mu$m. Mass median particle size is an important factor influencing absorption behavior of the water-absorbing resin or the water absorbing agent and finishing of absorbing articles such as a diaper, and thus problems cannot be solved merely by increasing mass median particle size to inhibit deterioration caused by urine.

Further, deterioration of the water absorbing agent caused by urine also correlates to absorption capacity and higher absorbency significantly increases extractables due to deterioration caused by urine. Therefore, it is not enough to increase only absorption capacity of the water-absorbing resin aiming at improving absorption amount of absorbing articles such as a diaper, to attain long term durability in practical use.

This indicates that, in a present day with favor of thinner type absorbing articles, such the water absorbing agent as having small mass median particle size D50 of not larger than 400 $\mu$m, high absorption capacity and certain limited range of extractables irrespective of change in urine can be a more superior water absorbing agent than conventional ones, however, further indicates that such the water absorbing agent as having small mass median particle size D50 of not larger than 400 $\mu$m, high absorption capacity and low extractables irrespective of change in urine is difficult to prepare practically and has thus not been present before.

By consideration of the above, the inventors thought of that the problems could be solved, in the present invention, by making the water absorbing agent with also superior stability to urine in specific mass median particle size, by furnishing crosslinked structure, specific centrifuge retention capacity, specific particle size distribution and mass median particle size and further specific ranges of "increased extractables by deterioration" and "increased ratio of extractables by deterioration" as a water absorbing agent.

The particulate water absorbing agent of the present invention is:

> a particulate water absorbing agent comprising a water-absorbing resin obtained by crosslinking polymerization of an unsaturated monomer containing an acid group and/or a salt thereof, wherein said water-absorbing resin is further surface crosslinked, and wherein said water absorbing agent satisfies the following (a) to (d) and (l):

>> (a) centrifuge retention capacity (CRC) of the water absorbing agent in a physiological saline solution being not lower than 32 g/g;
>> (b) mass median particle size (D50) of the water absorbing agent being in the range of 200 to 400 $\mu$m;
>> (c) ratio of particles of the water absorbing agent having diameter of smaller than 150 $\mu$m being in the range of 0 to 2 % by weight;
>> (d) increased extractables of the water absorbing agent by deterioration expressed by the following formula (I) of 0 to 15 % by weight and extractables of the water absorbing agent for one hour in deterioration test liquid of 0.1 to 30 % by weight, wherein the deterioration test liquid means a physiological saline solution containing 0.05% by weight of L-ascorbic acid;

formula (I):

Increased extractables by deterioration (% by weight)

= extractables for one hour in deterioration test liquid (% by weight)

- extractables for one hour in a physiological saline solution (% by weight);

> and
>> (l) logarithmic standard deviation of particle size distribution being in the range of 0.20 to 0.40.

In a preferred embodiment of the invention a particulate water absorbing agent satisfies further (e):

> (e) increased ratio of extractables of the water absorbing agent by deterioration expressed by the formula (II) of 1 to 4 times, and extractables of the water absorbing agent for one hour in deterioration test liquid of 0.1 to 30 % by weight, wherein the deterioration test liquid means a physiological saline solution containing 0.05% by weight of L-ascorbic acid;

formula (II):

Increased ratio of extractables by deterioration = extractables for one hour in deterioration test liquid (% by

weight)/extractables for one hour in a physiological saline solution

(% by weight).

In a further preferred embodiment of the invention a particulate water absorbing agent satisfies further the following (f) and (g):

(f) extractables of the water absorbing agent for 16 hours in a physiological saline solution being in the range of 0.1 to 10 % by weight; and
(g) absorbency against pressure at 4.8 kPa (AAP4.8 kPa) of the absorbent in a physiological saline solution being not lower than 21 g/g, preferably not lower than 22 g/g.

A method for production of the first particulate water absorbing agent of the present invention is characterized by comprising:

a step of crosslinking polymerization of the aqueous solution of an unsaturated monomer containing non-neutralized acrylic acid and/or salts thereof in the presence of a crosslinking agent and a chain transfer agent in an amount of 0.001 % to 1 % by mole based on total monumers;
a step of adjustment to particle size distribution;
a step of surface crosslinking the water-absorbing resin particle obtained by the polymerization and satisfying (a) to (c) described below,

(a) centrifuge retention capacity (CRC) of the resin particle in a physiological saline solution being in the range of not lower than 32 g/g;
(b) mass median particle size (D50) of the resin particle being in the range of 200 to 400 $\mu$m; and
(c) ratio of the particles having diameter of the resin particle of smaller than 150 $\mu$m being in the range of 0 to 2 % by weight.

A production method of the particulate water absorbing agent of the present invention is characterized by comprising:

a step of crosslinking polymerization of the aqueous solution of an unsaturated monomer in concentration of 10 to 30 % by weight containing non-neutralized acrylic acid in the presence of a crosslinking agent;
a step of neutralization after the polymerization; and
a step of surface crosslinking the water-absorbing resin particle obtained by the neutralization and satisfying (a) to (c) described below,

(a) centrifuge retention capacity (CRC) of the resin particle in a physiological saline solution being in the range of not lower than 32 g/g;
(b) mass median particle size (D50) of the resin particle being in the range of 200 to 400 $\mu$m; and
(c) ratio of particles having diameter of the resin particle of smaller than 150 $\mu$m being in the range of 0 to 2 % by weight.

A production method of the particulate water absorbing agent of the present invention is characterized by comprising:

a step of crosslinking polymerization of the aqueous solution of an unsaturated monomer containing non-neutralized acrylic acid and/or salts thereof as a main component in the presence of a crosslinking agent;

a step of surface crosslinking the water-absorbing resin particle obtained by the polymerization and satisfied (a) to (c) described below,

(a) centrifuge retention capacity (CRC) of the resin particle in a physiological saline solution being in the range of not lower than 32 g/g;
(b) mass median particle size (D50) of the resin particle being in the range of 200 to 400 $\mu$m;
(c) ratio of particles having diameter of the resin particle of smaller than 150 $\mu$m being in the range of 0 to 2% by weight; and

a step of adding a chelating agent with one or more timings selected from the group consisting of

(i) during polymerization
(ii) after the polymerization and before surface crosslinking
(iii) during surface crosslinking
(iv) after surface crosslinking.

Effects of the Invention

Using the particulate water absorbing agent of the present invention, due to having specific absorption capacity and specific particle size distribution, in practical application as absorbing articles such as a diaper, particularly in absorbing ability in short period, not conventionally obtained performance can be attained. In particular, rewet amount can be reduced and improvement effect of dry feeling at diaper surface is significant.

Further, due to inhibition of deterioration caused by urine, it provides superior gel stability and maintains absorbing performance over long period, therefore it reduces discomfort for persons wearing absorbing articles.

In the present invention, because deterioration caused by urine can be inhibited and simultaneously specific particle size distribution is provided, size segregation is less and in powder transfer to produce the particulate water absorbing agent and produce absorbing articles such as a diaper, superior piston-flow property is provided and pulsation which is periodical change in powder feed amount is inhibited. In addition to this, in production of absorbing articles such as a diaper, mixing is easy between the particulate water absorbing agent of the present invention and hydrophilic fibers such as wood crushed pulp, which conveniently provides homogeneous composition.

Best embodiments to practice the Invention

Raw materials used for the water-absorbing resin and the water absorbing agent of the present invention and reaction conditions will be explained below. In the present invention, the followings are values obtained by methods described in Examples shown later: (a) centrifuge retention capacity (CRC) in a physiological saline solution, (b) mass median particle size (D50), (d) increased extractables by deterioration, (e) increased ratio of extractables by deterioration, (f) extractables for 16 hours in a physiological saline solution, (g) absorbency against pressure at 4.8 kPa (AAP4.8 kPa) in a physiological saline solution, (i) absorbency against pressure at 1.9 kPa (AAP1.9 kPa) in a physiological saline solution, (j) absorption speed with vortex method in a physiological saline solution, (k) fluidity after moisture absorption, (1) logarithmic standard deviation of particle size distribution and extractables for one hour in deterioration test liquid.

(1) A water-absorbing resin

A water-absorbing resin of the present invention means a crosslinked polymer which can form hydrogel and is water swelling and non-dissolving in water, for example, water swelling indicates one absorbing large quantity of water in ion exchanged water, such as essentially 5 times or more own weight and preferably 50 to 1000 times. Non-dissolving in water means one with extractables in water in 1 hour of not higher than 50% by weight and in the range described later. Measurement methods thereof are specified in Examples.

As the water-absorbing resin in the present invention, to attain objectives of the present invention, a water-absorbing resin obtained by crosslinking polymerization of an unsaturated monomer containing an acid group and/or salts thereof is essentially used and preferably (partially) neutralized polymer of polyacrylic acid obtained by polymerizing and crosslinking of an unsaturated monomer mainly composed of acrylic acid and/or salts thereof is used. Any water-absorbing resin may be used as long as it has crosslinked polymerized structure and it may be the water-absorbing resin obtained by crosslinking reaction with a crosslinking agent after polymerization of an unsaturated monomer containing an acid group and/or salts thereof.

(2) A water absorbing agent and a method for production thereof

A water absorbing agent in the present invention is a solidifying agent made of the water-absorbing resin as a main component, to absorb aqueous liquid. Aqueous liquid is not limited to water but also includes water containing substance without especially limited, such as urine, blood, excrement, waste liquid, humidity or steam, ice, a mixture of water and organic solvents or inorganic solvents, rain water and underground water, preferably urine and particularly preferably human urine. In the present invention, the water-absorbing resin may be used as it is as the water absorbing agent and additives or water may be contained optionally. Content of the water-absorbing resin in the water absorbing agent is 70 to 100% by weight of the water absorbing agent, preferably 80 to 100% by weight and further preferably 90 to 100% by weight. As other components contained, generally water is used as a main or essential component and further additives described later are used.

A water absorbing agent of the present invention can be obtained, for example, by the following production methods 1 to 3, although a method for production is not limited as long as it provides one satisfying the properties.

Production method 1: A method for crosslinking polymerization of an aqueous solution of an unsaturated monomer containing non-neutralized acrylic acid and/or salts thereof as a main component in the presence of a crosslinking agent and a chain transfer agent, followed by adjustment to specific particle size distribution and further surface crosslinking ofthusobtained water-absorbing resin particles with specific absorption capacity.

Production method 2: A method for crosslinking polymerization of an aqueous solution of specific concentration of an unsaturated monomer containing non-neutralized acrylic acid as a main component in the presence of a crosslinking agent, followed by neutralization, adjustment to specific particle size distribution and further surface crosslinking of thus obtained water-absorbing resin particles with specific absorption capacity.

Production method 3: A method for crosslinking polymerization of an aqueous solution of an unsaturated monomer containing non-neutralized acrylic acid and/or salts thereof as a main component in the presence of a crosslinking agent, followed by adjustment to specific particle size distribution and further surface crosslinking of thus obtained water-absorbing resin particles with specific absorption capacity, wherein a chelating agent is added with one or more timings selected from the group consisting of

(i) during polymerization
(ii) after the polymerization and before surface crosslinking
(iii) during surface crosslinking
(iv) after surface crosslinking.

A production method for the water absorbing agent of the present invention and further the water absorbing agent of the present invention are explained below sequentially.

(3) An unsaturated monomer

As an unsaturated monomer composing the water-absorbing resin (herein after abbreviated simply as a monomer), acrylic acid and/or salt thereof is used as a main component, and they may be used alone or in combination with other monomers to obtain the water-absorbing resin. Such other monomers include, an aqueous or hydrophobic unsaturated monomer such as methacrylic acid, maleic anhydride, maleic acid, fumaric acid, crotonic acid, itaconic acid, vinylsulfonic acid, 2-(meth)acrylamide-2-methylpropane sulfonic acid, (meth) acryloxyalkane sulfonic acid and its alkali metal salt, ammonium salt, N-vinyl-2-pyrrolidone, N-vinylacetamide, (meth)acrylamide, N-isopropyl (meth)acrylamide, N,N-dimethyl(meth)acrylamide, 2-hydroxyethyl (meth)acrylate, methoxypolyethyleneglycol (meth)acrylate, polyethyleneglycol (meth)acrylate, isobutylene, lauryl (meth) acrylate, etc. They may be used alone or in combination of two or more kinds.

When monomers other than acrylic acid (salts) are used in combination, to attain obj ectives of the present invention, use ratio of the monomer other than acrylic acid (salt thereof) is preferably 0 to 30% by mole based on total amount of acrylic acid and salts thereof, more preferably 0 to 10% by mole and most preferably 0 to 5% by mole.

When an unsaturated monomer containing an acid group is usedas a monomer, salts thereof include alkali metal salts, alkaline earth metal salts and ammonium salts, in view of performance, industrial availability and safety of the water-absorbing resin obtained, sodium salts and potassium salts are preferable. An unsaturated monomer containing an acid group such as acrylic acid is preferably neutralized at the acid group in view of property and pH and neutralization ratio of the acidgroup is usually 20 to 100% by mole, preferably 30 to 95% by mole and more preferably 40 to 80% by mole. Neutralization of the acid group maybe performed in an aqueous solution containing a monomer or may be performed after obtaining a polymer as shown in the production method 2 or they may be used in combination.

(4) An internal crosslinking agent

A water-absorbing resin used in the present invention is a crosslinked polymer and crosslinked structure may be formed as self-crosslinked type without using a crosslinkable monomer or an internal crosslinking agent such as so called a crosslinkable monomer may be used. In view of property, it is preferable to copolymerize or react with an internal crosslinking agent having not less than 2 polymerizable unsaturated groups or not less than 2 reactable groups in a molecule. A water absorbing agent becomes insoluble to water due to being a crosslinked polymer.

Specific examples of these internal crosslinking agents include, for example, N,N'-methylenebis(meth)acrylamide, (poly)ethyleneglycol di(meth)acrylate, (poly)propyleneglycol di(meth)acrylate, trimethylolpropane tri(meth)acrylate, glycerine tri(meth)acrylate, glycerine acrylate methacrylate, ethylene oxide modified trimethylolpropane tri(meth)acrylate, pentaerythritol tetra(meth)acrylate, triallyl cyanurate, triallyl isocyanurate, triallyl phosphate, triallylamine, poly(meth)allyloxyalkane, (poly)ethyleneglycol diglycidyl ether, glycerol diglycidyl ether, ethyleneglycol, polyethyleneglycol, propyleneglycol, glycerine, pentaerythritol, ethylenediamine, ethylene carbonate, propylene carbonate, polyethyleneimine, glycidyl (meth)acrylate, etc.

These internal crosslinking agents may be used alone or in a mixture of two or more kinds, as appropriate. These internal crosslinking agents may be added as a whole into a reaction system or in portion wise. When at least one kind or not less than 2 kinds of internal crosslinking agents are used, in consideration of absorption properties of the water-absorbing resin or the water absorbing agent finally obtained, it is preferable to use essentially a compound having not less than two polymerizable unsaturated groups, in polymerization.

Using amount of these internal crosslinking agents is preferably in the range of 0.001 to 2% by mole based on the unsaturated monomer (excluding the internal crosslinking agents), more preferably 0.005 to 0.5% by mole, further preferably 0.01 to 0.2% by mole and particularly preferably 0.03 to 0.15% by mole. The using amounts of the internal crosslinking agents less than 0.001% by mole and over 2% by mole may not provide sufficient absorption properties. The using amount of the internal crosslinking agents less than the range does not sufficiently form crosslinked structure, which increases extractables in a physiological saline solution or extractables in deterioration test liquid described later, increased extractables by deterioration, increased ratio of extractables by deterioration and "extractables for 16 hours" and thus not preferable. On the other hand, the using amount of the internal crosslinking agents over the range reduces the extractables, however, induces reduced absorption capacity of the water-absorbing resin or the water absorbing agent, which in turn lowers absorption capacity of absorbing articles such as a diaper, and thus not preferable.

When crosslinked structure is introduced inside a polymer by using the internal crosslinking agent, the internal crosslinking agent may be added to a reaction system before, during or after polymerization of the monomer or after neutralization.

(5) A polymerization initiator

An initiator used in polymerization of a monomer to obtain the water-absorbing resin used in the present invention includes a radical polymerization initiator such as potassium persulfate, ammonium persulfate, sodium persulfate, potassium peracetate, sodium peracetate, potassium percarbonate, sodium percarbonate, tert-butylhydroperoxide, hydrogen peroxide, 2,2'-azobis(2-amidinopropane) dihydrochloride : photopolymerization initiators such as 2-hydroxy-2-methyl-1-phenylpropane-1-one. Using amount of the polymerization initiator is, in view of property, 0.001 to 2% by mole, preferably 0.01 to 0.1% by mole (based on total monomers). When the using amount of the polymerization initiator is less than 0.001% by mole, unreacted residual monomers increase, while, the amount of the polymerization initiator is over 2% by mole, polymerization control becomes difficult and thus not preferable.

(6) A polymerization method

In the present invention, bulk polymerization or precipitation polymerization may be carried out, however, in view of property, aqueous solution polymerization or reversed phase suspension polymerization by preparation of an aqueous solution of the monomer is preferable. Monomer concentration in the aqueous solution (hereinafter referred to as a monomer aqueous solution), when an aqueous solution of the monomer is prepared, is determined by temperature of the aqueous solution or the monomer and not especially limited, however, preferably 10 to 70% by weight, and further preferably 20 to 60% by weight. When aqueous solution polymerization is carried out, a solvent other than water may also be used, if necessary, and solvent type used in combination is not especially limited. Crushing may be carried out after polymerization, if necessary.

Polymerization is started using the polymerization initiator. An activated energy ray such as UV ray, electron beam or γ-ray may be used other than the polymerization initiator as it is or in combination with the polymerization initiator. Polymerization temperature depends on type of the polymerization initiator used, however, is preferably in the range of 15 to 130°C and more preferably in the range of 20 to 120°C.

Reversed phase polymerization is a method for polymerization by suspending a monomer aqueous solution in a hydrophobic organic solvent, and described, for example, in US4093776, US4367323, US4446261, US4683274, US5244735, etc. Aqueous solution polymerization is the method for polymerization of a monomer aqueous solution without using a dispersing solvent, and described, for example, in US4625001, US4873299, US4286082, US4973632, US4985518, US5124416, US5250640, US5264495, US5145906, US5380808, EP0811636, EP0955086, EP0922717, etc. Monomers or polymerization initiators exemplified in these polymerization methods are also applicable to the present invention.

A water absorbing agent of the present invention has, as described above, neutralization degree of acid groups of generally 20 to 100% by mole, but in a polymerization process of an unsaturated monomer, the unsaturated monomer may be polymerized as not-neutralized state and neutralized after polymerization, or polymerization may be carried out using the unsaturated monomer neutralized in advance. Therefore, neutralization degree of the unsaturated monomer in the monomer aqueous solution may be in any range of 0 to 100% by mole. Among these, in the production method 1 or the production method 3 may also be neutralization polymerization and polymerization can be carried out using the monomer aqueous solution with neutralization degree of 20 to 100% by mole, preferably 30 to 95% by mole and more preferably 40 to 80% by mole. Neutralization embodiments include to start polymerization using the non-neutralized unsaturated monomer, followed by neutralization in the midst of polymerization; to polymerize using the unsaturated monomer neutralized in advance to the above range; and to neutralize further in the midst of polymerization, all of which providing polymerization of the unsaturated monomer neutralized finally, and the neutralization degree means value at the start of polymerization.

On the other hand, so to speak a method for acid polymerization, followed by neutralization may be adopted, wherein the non-neutralized unsaturated monomer containing an acid group, in particular, non-neutralized acrylic acid as amain component is polymerized, followed by neutralization of the acid group. This corresponds to the production method 2. That is, the production method 2 of the present invention is a method for crosslinking polymerization of specific concentration of the unsaturated monomer aqueous solution with non-neutralized acrylic acid as a main component in the presence of a crosslinking agent, followed by neutralization, adjustment to specific particle size and further surface crosslinking of thus obtained water-absorbing resin particles with specific absorption capacity. In the production method 2, non-neutralized acrylic acid is a main component and after crosslinking polymerization using a non-neutralized acrylic acid monomer in the range of preferably 30 to 100% by mole, more preferably 90 to 100% by mole and particularly preferably 100% by mole, followed by the addition of an alkali metal salt for post neutralization to provide partial alkali metal base to be used as a water-absorbing resin of the present invention. When the water-absorbing resin obtained by this polymerization method is used as a water absorbing agent of the present invention, it is possible to obtain an absorbing substrate with high absorbing ability and superior stability to urine. When the non-neutralized unsaturated monomer is polymerized, using amount of an internal crosslinking agent tends to be able to increase, although details are unknown, and deterioration resistance to urine can be improved by increase in crosslinking density.

In the present invention, other polymerizable monomers can be used with acrylic acid, if necessary. Specific other polymerizable monomers, internal crosslinking agents, types of polymerization initiators, additives, and the like are the same as described in the content of the items (3), (4) and (5). In the production method 2, concentration of the polymerizable monomer, when a solvent is used, is not especially limited, however, is as low as generally 5 to 30% by weight and preferably 10 to 30% by weight and polymerization is preferably carried out in an aqueous solution at temperature as low as 10 to 25°C.

Alkali metal compounds used to neutralize an acid group in the unsaturated monomer containing an acid group or in the polymer obtained to provide a partial alkali metal base include alkali metal hydroxide (sodium hydroxide, potassium hydroxide, lithium hydroxide, etc.), alkali metal carbonate (sodium carbonate, potassium bicarbonate, etc.), etc. In view of performance, industrial availability and safety of the water-absorbing resin obtained, sodium salts and potassium salts are preferable among them. In the present invention, 50 to 90% by mole, preferably 60 to 80% by mole of acid groups in the polymer are converted to alkali metal salts by neutralization reaction with an alkali metal compound.

In the production method 2, the polymer after polymerization is essentially neutralized. A method for neutralization of the polymer with an alkali metal compound includes, when polymerization is carried out using a solvent, one wherein an aqueous solution of an alkali metal compound is added, while cutting the gel-like polymer obtained to small pieces of not larger than about 1 cm$^3$, followed by further mixing the gel with a kneader or a meat chopper. Neutralization temperature to obtain the water absorbing agent of the present invention is 50 to 100°C, preferably 60 to 90°C and neutralization is preferably performed so that uniformly of not larger than 10 as represented by the first neutralization index (specified by neutralization degree of 200 particles) described in claim 1 of US6187872.

(7) A chain transfer agent

In the present invention, a chain transfer agent may be used essentially in polymerization. By polymerization in the presence of an aqueous chain transfer agent in addition to the unsaturated monomer, inner crosslinking agent and polymerization initiator, and when the water-absorbing resin thus obtained is used as the water absorbing agent of the present invention, an absorbing substrate with high absorbing ability and superior stability to urine can be obtained. When the chain transfer agent is used in combination, using amount of the inner crosslinking agent can be increased and deterioration resistance to urine can be improved by increase in crosslinking density. The aqueous chain transfer agent used for polymerization in the present invention is not especially limited as long as it dissolves in water or an aqueous ethylenic unsaturated monomer and includes thiols, thiolates, secondary alcohols, amines, phosphites, hypophosphites, etc. Specifically, mercaptoethanol, mercaptopropanol, dodecylmercaptan, thioglycols, thiomalic acid, 3-mercaptopropionic acid, isopropanol, sodium phosphite, potassium phosphite, sodium hypophosphite, formic acid and their salts are used, and one kind or not less than 2 kinds selected from the group can be used. In view of the effect, phosphorous compounds, in particular, hypophosphite salts such as sodium hypophosphite are preferably used.

Using amount of the aqueous chain transfer agent depends on kind of the aqueous chain transfer agent and concentration of a monomer aqueous solution, however, is 0.001 to 1% by mole based on total monomers and preferably 0.005 to 0.3% by mole. The using amount less than 0.001% by mole provides high crosslinking density under using amount of the inner crosslinking agent in the present invention and too low absorption capacity and thus not preferable. On the other hand, the using amount over 1% by mole increases water extractables and lowers stability on the contrary and thus not preferable. The chain transfer agent may be added by dissolving in the monomer aqueous solution before polymerization or sequentially in the midst of polymerization. The aqueous chain transfer agent is essential in the production method 1 and it may also be used in the production method 2 and the production method 3.

(8) Drying

A crosslinked polymer obtained by the above polymerization methods is the hydrated gel-like crosslinked polymer, whichmaybe crushed, if necessary, and further dried. Drying is carried out generally at temperature range of 60 to 250°C, preferably 100 to 220°C and more preferably 120 to 200°C as heating medium temperature. Drying time depends on surface area and water content of the polymer and the dryer type and is selected to obtain objective water content. In the present invention, the crosslinked polymer after drying is called the water-absorbing resin.

Water content of the water-absorbing resin used in the present invention is not especially limited, however, it is selected so as to provide a particle exhibiting fluidity even at room temperature and powder state with water content of more preferably 0.2 to 30% by weight, further-preferably 0.3 to 15% by weight and particularly preferably 0.5 to 10% by weight. Too high water content not only impairs fluidity and thus affects production but also makes pulverizing of the water-absorbing resin impossible and may lose control to specific particle size distribution. Water content of the water-absorbing resin is specified as amount of water contained in the water-absorbing resin, measured by weight loss in drying at 180°C for 3 hours.

As a drying method used, various methods can be adopted, so that objective water content is obtained, including heat drying, hot air drying, reduced pressure drying, infrared ray drying, microwave drying, dehydration by azeotrope with a hydrophobic organic solvent and high-humidity drying using high temperature steam, however, not especially limited.

Shape of the water-absorbing resin of the present invention obtained by the above production methods is not especially limited, as long as it is suitable to be treated as powder and includes spherical, fibrous, rod, nearly spherical, flat, irregular, agglomelated particulate, porous-structured particles, however, irregularly pulverized one obtained by a pulverizing process is preferably used.

(9) Pulverizing, classification and particle size control and absorption capacity

A water-absorbing resin used in the present invention is adjustedpreferably to have specific particle size to obtain the particulate water absorbing agent of the present invention.

Particle diameter of the water-absorbing resin used in the present invention to obtain the water absorbing agent of the present invention is usually controlled in small range of 180 to 420 $\mu$m, as mass median particle size, preferably 200 to 400 $\mu$m, more preferably 225 to 380 $\mu$m and particularly preferably 250 to 350 $\mu$m, and ratio of particles with diameter of lower than 150 $\mu$m is controlled to be 0 to 3% by weight, preferably 0 to 2% by weight and more preferably 0 to 1% by weight.

Bulk density (specified by JIS K-3362-1998) of the water-absorbing resin of the present invention to obtain the water absorbing agent of the present invention is adjusted to be in the range of preferably 0.40 to 0.90 g/ml and more preferably 0. 50 to 0. 80 g/ml. Ratio of particles with diameter of 150 to 600 $\mu$m is preferably 90 to 100% by weight in whole particles, more preferably 95 to 100% by weight and further more preferably 98 to 100% by weight. Logarithmic standard deviation ($\sigma\zeta$) of particle size distribution is controlled preferably to be 0.20 to 0.50, more preferably 0.20 to 0.45 and particularly preferably 0.20 to 0.40.

Particle size may be adjusted, when crosslinked polymer is produced by reversed phase suspension polymerization, by dispersion polymerization in particulate state and distribution drying, however, it is generally adjusted, particularly in an aqueous solution polymerization, by pulverizing and classification after drying, while controlling mass median particle size D50 and ratio of particles having particle diameter of below 150 $\mu$m, being mutually contradictory, and thus adjusting to specific particle size. For example, in the adjustment to specific particle size to obtain mass median particle size D50 of not larger than 400 $\mu$m and to reduce content of fine particles with diameter smaller than 150 $\mu$m, coarse particles and fine particles may be removed using a general classifier such as a sieve after the pulverizing. In that case, the coarse particles to be removed have particle diameter of preferably 400 $\mu$m to 5000 $\mu$m, more preferably 400 $\mu$m to 2000 $\mu$m, further preferably 400 $\mu$m to 1000 $\mu$m. On the other hand, the fine particles to be removed by the particle size adjustment have particle diameter of preferably smaller than 150 $\mu$m and more preferably smaller than 200 $\mu$m. Thus removed coarse particles and fine particles may be disposed as they are. The coarse particles, however, are preferably pulverized again by the pulverizing process. While, the fine particles removed are subjected to particle size enlargement process mentioned at the following item (10) to reduce loss.

A water-absorbing resin thus obtained in the present invention is adjusted to have the above particle size and preferably the centrifuge retention capacity (CRC) in a physiological saline solution before surface crosslinking is controlled to be not lower than 32 g/g, more preferably 35 to 70 g/g, further preferably 40 to 65 g/g and particularly preferably 45 to 60 g/g. "The centrifuge retention capacity" can be controlled by using specific amount of an inner crosslinking agent to an aqueous solution of an unsaturated monomer or by controlling the polymerization conditions or drying conditions. A water-absorbing resin can be used as the water absorbing agent as it is and the centrifuge retention capacity of the water-absorbing resin is also required to be not lower than 32 g/g to obtain the water absorbing agent of the present invention having the centrifuge retention capacity of not lower than 32 g/g.

(10) Diameter enlargement of fine particles

The fine particles removed in pulverizing, classification and particle size control in the item (9) are regenerated to larger particles or particulate aggregates to be used as the water-absorbing resin of the present invention. In this case, methods described in US6228930, US5264495, US4950692, US5478879 and EP844270 can be used, and, thus regenerated water-absorbing resin has substantially porous structure.

Ratio of the regenerated water-absorbing resin by this process, contained in the water-absorbing resin of the present invention is preferably 0 to 50% by weight, more preferably 5 to 40% by weight and most preferably 10 to 30% by weight. The regenerated water-absorbing resin by this process, when used as the water-absorbing resin of the present invention, has larger surface area than a non-regenerated one and provides higher absorption speed, thus advantageous in view of performance. A water-absorbing resin with thus enlarged particle diameter is generally subjected to pulverizing, classification and particle size control after mixing with the water-absorbing resin obtained by the drying process (8).

(11) Surface crosslinking treatment

A water-absorbing resin of the present invention may be, as represented by the production methods 1 to 3, one with specific absorption capacity obtained by adjustment to specific particle size distribution, followed by further surface crosslinking. A water-absorbing resin used in the present invention has the centrifuge retention capacity (CRC) lowered by such surface crosslinking, for example, to generally 50 to 95% of the centrifuge retention capacity (CRC) before surface crosslinking and further to 60 to 90%. Lowering of the centrifuge retention capacity can be adjusted by type and amount of the crosslinking agent, reaction temperature and time, as appropriate.

A surface crosslinking agent used in the present invention is not especially limited, however, for example, those exemplified in US6228930, US6071976, US6254990, and the like can be used and includes, for example, polyhydric alcohols such as mono, di-, tri-, tetra- or polyethyleneglycol, monopropyleneglycol, 1,3-propanediol, dipropyleneglycol, 2,3,4-trimethyl-1,3-pentanediol, polypropyleneglycol, glycerin, polyglycerin, 2-butene-1,4-diol, 1,4-butanediol, 1,3-butanediol, 1,5-pentanediol, 1,6-hexanediol, 1,2-cyclohexanedimethanol, etc.; epoxy compounds such as ethyleneglycol diglycidyl ether, glycidol, etc.; polyvalent amine compounds such as ethylenediamine, diethylenetriamine, triethylenetetramine, tetraethylenepentamine, pentaethylenehexamine, polyethyleneimine, polyamidepolyamine, etc.; haloepoxy compounds such as epichlorohydrin, epibromohydrin, $\alpha$-methylepichlorohydrin, etc.; condensates of the polyvalent amine compounds and the haloepoxy compounds; oxazolidinone compounds such as 2-oxazolidinone, etc.; cyclic urea; alkylene carbonate compounds such as ethylene carbonate, etc. They may be used alone or in combination of two or more kinds. To sufficiently exert effects of the present invention, it is preferable to essentially use a polyvalent alcohol among these surface crosslinking agents. As a polyvalent alcohol, one having carbon atoms of 2 to 10 is preferable and one having carbon atoms of 3 to 8 is more preferable.

Using amount of the surface crosslinking agent depends on compounds used or combination thereof, however, is preferably in the range of 0.001 to 10% by weight based on the water-absorbing resin and more preferably in the range of 0.01 to 5% by weight.

In surface crosslinking in the present invention, water is preferably used as a solvent. In this case, using amount of water depends on water content of the water-absorbing resin used, however, is preferably in the range of 0.5 to 20% by weight based on the water-absorbing resin and more preferably in the range of 0.5 to 10% by weight. A hydrophilic organic solvent other than water may be used. When a hydrophilic organic solvent is used, using amount thereof is preferably in the range of 0 to 10% by weight based on the water-absorbing resin, more preferably in the range of 0 to 5% by weight and further preferably in the range of 0 to 3% by weight.

In surface crosslinking in the present invention, the preferable method is premixing of a surface crosslinking agent in water and/or a hydrophilic organic solvent, followed by spraying or drop-wise addition of the solution to the water-absorbing resin and the spraying method is more preferable. Droplet size to be sprayed is, as average particle diameter, preferably in the range of 0.1 to 300 μm and more preferably in the range of 0.1 to 200 μm.

Mixing equipment to be used in mixing the water-absorbing resin, the crosslinking agent and water or the hydrophilic organic solvent is preferably one with strong mixing force to uniformly and surely mix them. Suitable mixing equipment includes, for example, a cylinder type mixer, a double wall conical mixer, a high speed agitation type mixer, a V-shaped mixer, a ribbon type mixer, a screw type mixer, a double-arm kneader, a crushing type kneader, a rotation type mixer, an air-flow type mixer, a turbulizer, a batch type Lödige mixer, a continuous type Lödige mixer, etc.

A water-absorbing resin after the addition of the surface crosslinking agent is preferably subjected to heat treatment. Heating temperature (temperature of heating medium or temperature of material) is preferably in the range of 100 to 250°C, more preferably in the range of 150 to 250°C and heating time is preferably in the range of 1 minute to 2 hours. A suitable combination example of heating temperature and heating time is 180°C for 0.1 to 1.5 hour and 200°C for 0.1 to 1 hour. A particulate water-absorbing resin can be obtained by these processes.

(12) Agglomeration

A water-absorbing resin used in the present invention may further be agglomerated in addition to the above processes. Agglomeration process includes the addition of aqueous liquid to water-absorbing resin after surface crosslinking treatment, then the heating with maintaining water content of 1 to 10% by weight, and further if necessary the adjusting to specific particle size of obtained water-absorbing resin by Pulverizing and classification mentioned above.

In the present invention, agglomerating is preferably performed by a method for spraying or drop-wisely addition of water or an aqueous solution dissolved with other additives components to the water-absorbing resin, and, in particular, a spraying method is preferable. Droplet size to be sprayed is, as average particle diameter, preferably in the range of 0.1 to 300 μm and more preferably in the range of 0.1 to 200 μm. Heat treatment is performed, in view of agglomerating ratio or agglomerating strength, by maintaining water content (specified by weight loss in drying at 180°C for 3 hours) of the water-absorbing resin, at 1 to 10% by weight, more preferably at 2 to 8% by weight and further preferably at 2.5 to 6% by weight. Heating medium such as hot air can be used in heating and heating temperature (temperature of heating medium or temperature of material) is preferably in the range of 40 to 120°C, more preferably in the range of 50 to 100°C and heating time is preferably in the range of 1 minute to 2 hours. Heating temperature is expressed by temperature of heating medium in many cases. A suitable combination example of heating temperature and heating time is 60°C for 0.1 to 1.5 hour and 100°C for 0.1 to 1 hour. Heating and the addition of water may be carried out by the same equipment or by separate equipment. Heating may be carried out, with stirring or without stirring, as long as temperature or water content can be controlled, but heating without stirring is preferable. A more preferable method is heating of the water-absorbing resin added with water, and the like by heaping up of 1 to 100 cm, more preferably 5 to 80 cm and particularly preferably 10 to 70 cm. A hardened water-absorbing resin can be obtained by heating and the particulate water-absorbing resin can be obtained by subsequent crushing and classification.

As agglomerating equipment to be used, those having strong mixing force are preferable, including a cylinder type mixer, a double wall conical mixer, a high speed agitation type mixer, a V-shaped mixer, a ribbon type mixer, a screw type mixer, a double-arm kneader, a crashing type kneader, a rotation type mixer, an air-flow type mixer, a turbulizer, a batch type Lödige mixer, a continuous type Lödige mixer, etc.

Water to be added to the water-absorbing resin may contain other additives such as chelating agents described later, components made fromplants, antimicrobials, aqueous polymers, inorganic salts, etc. Content of the additives is in the range of 0.001 to 50% by weight in an aqueous solution.

(13) Addition of a chelating agent

To the particulate water absorbing agent of the present invention, a chelating agent, in particular, a polyvalent carboxylic acid and salts thereof can be formulated.

The production method 3 of the present invention, in particular, is a method for crosslinking polymerization of an aqueous solution of an unsaturated monomer with non-neutralized acrylic acid and/or salts thereof, as main components, in the presence of a crosslinking agent, followed by adjustment to specific particle size distribution and further surface crosslinking of thusobtained water-absorbing resin particles with specific absorption capacity, wherein the chelating agent is added during polymerization or, before or after surface crosslinking. By the addition effect of the chelating agent, it is possible to inhibit action of a component to deteriorate or destruct crosslinked structure and thus to produce the water absorbing agent superior in deterioration resistance to urine.

The chelating agent used in the water absorbing agent of the present invention is preferably one with high blocking ability or chelating ability for Fe or Cu ion, specifically, one with stability constant for Fe ion of not lower than 10, preferably not lower than 20, further preferably an aminopolyvalentcarboxylic acid and salts thereof and particularly preferably aminocarboxylic acid with not less than 3 carboxyl groups and salts thereof. Among these, aminocarboxylate salts may be those, wherein a part of the acid groups contained is neutralized or all acid groups are neutralized. These polyvalent carboxylic acids specifically include diethylenetriamine pentaacetic acid, triethylenetetramine hexaacetic acid, cyclohexane-1,2-diamine tetraacetic acid, N-hydroxyethyl ethylenediamine triacetic acid, ethyleneglycol diethylether diamine tetraacetic acid, ethylenediamine tetra propionic acetic acid, N-alkyl-N'-carboxymethyl aspartic acid, N-alkenyl-N'-carboxymethyl aspartic acid and alkaline metal salts thereof; alkaline earth metal salts; ammonium salts or amine salts. They may be used alone or in combination of two or more kinds. Among these, diethylenetriamine pentaacetic acid, triethylenetetramine hexaacetic acid, N-hydroxyethyl ethylenediamine triacetic acid and salts thereof are most preferable.

Using amount of the chelating agent, in particular, the amino polyvalent carboxylic acid is as small as generally 0.00001 to 10 parts by weight based on 100 weight parts of the water-absorbing resin, a main component, and preferably 0.0001 to 1 weight parts. The using amount over 10 parts by weight is not only uneconomical due to failure to get enough effect relative to using amount but also poses a problem of reducing absorption capacity. On the other hand, the using amount less than 0.00001 part by weight does not provide sufficient addition effect.

In the case of the addition of the chelating agent during polymerization, the chelating agent may be added to an aqueous solution of an unsaturated monomer, followed by polymerization or it maybe added in the midst of polymerization, or it may be added to the gel-like crosslinked polymer or the water-absorbing resin obtained. To add the chelating agent during surface crosslinking, surface crosslinking may be performed using a solution containing the surface crosslinking agent added with the chelating agent. Further, in the case of adding the chelating agent after surface crosslinking, it may be added to the water-absorbing resin after crosslinking. Further in the case of performing the agglomerating process of the above item (12), water dissolved with the chelating agent may be sprayed, followed by heating, while maintaining water content at 1 to 10% by weight, as described above. In the case of drying after the addition, if necessary, the temperature may be in the range not impair the effects of the chelating agent and generally in the range described in agglomerating process of the item (12). The chelating agent is essential in the production method 3, however, it may also be used to the water-absorbing resin obtained in the production method 1 and the production method 2.

(14) Other additives

In the present invention, the following (A) components made from plants, (B) polyvalent metal salts of organic acids, (C) inorganic fine particles (including (D) composite hydrated oxides) may be added as minor components in addition to the chelating agent, by which various functions can be added to the water-absorbing resin of the present invention. The addition methods include, in the case that the additives are solutions, an embodiment to add as a solution, as a water dispersion or as it is; while in the case that the additives are powders, non-soluble in water, an embodiment to add as a water dispersion or as it is; and in the case that the additives are soluble in water, the same embodiments as in the case of the solutions.

Using amount of these (A) to (D) and (E) other additives depends on objectives or function to be added, however, is usually, as the amount of one kind of additive, 0 to 10 parts by weight based on 100 parts by weight of the water-absorbing resin, preferably 0.001 to 5 parts by weight and further preferably 0.002 to 3 parts by weight. The using amount less than 0.001 part by weight usually does not provide sufficient effect or additional function, while the using amount over 10 part by weight may not get effect matching to the added amount or may incur lowering of absorption performance.

(A) Components made from plants

A water absorbing agent of the present invention can be added with components made from plants in the amount described above to fulfill deodorization effect. The components made from plants to be used in the present invention are preferably at least one compound selected frompolyphenol, flavone, derivatives thereof and caffeine. It is further preferable that the component made of plant is at least one kind selected from tannin, tannic acid, stachyurus praecox, gall or gallic acid.

Plants containing the components made from plants to be used in the present invention include, for example, Theaceae plant such as camellia, Hikasaki plant and Sprague; Gramineae plant such as rice plant, sasa-bamboo, bamboo, corn, wheet, etc.; and Rubiaceae plant such as coffee.

Form of the components made from plants to be used in the present invention includes plant extract (essential oil), plat itself (plant-ground powder), plant residue or extract residue as by-products in production processes from plant processing industry or foods processing industry, however, not limited thereto.

(B) A polyvalent metal salt

A water absorbing agent of the present invention may be added a polyvalent metal salt, in particular, a polyvalent metal salt of organic acids in the amount described above to improve powder fluidity and to maintain the fluidity after moisture absorption.

A polyvalent metal salt of an organic acid used and a methods for mixing the polyvalent metal salts are exemplified in WO PCT/2004/JP1355, and the polyvalent metal salt of organic acids used in the present invention, having carbon atoms of not less than 7 in a molecule include polyvalent metal salts of fatty acid, petroleum acid or polymeric acid, specifically calcium, aluminium, magnesium or zinc salts. They may be used alone or in combination of two or more kinds.

An organic acid composing the polyvalent metal salt of an organic acid exemplified are long chain or branched fatty acids such as caproic acid, octylic acid, octynoic acid, decanoic acid, lauric acid, myristic acid, palmitic acid, oleic acid, stearic acid, etc.; petroleum acids such as benzoic acid, myristicinic acid, naphthenic acid, naphthoic acid, naphthoxyacetic acid, etc.; polymeric acids such as poly (meth) acrylic acid, polysulfonic acid, and the like and preferable ones have a carboxyl group in a molecule and more preferably includes fatty acids such as caproic acid, octylic acid, octynoic acid, decanoic acid, lauric acid, myristic acid, palmitic acid, oleic acid, stearic acid, tallow acid or hydrogenated fatty acid of castor oil, etc. Further preferably, they are fatty acids without an unsaturated bond in a molecule, for example, caproic acid, octylic acid, decanoic acid, lauric acid, myristic acid, palmitic acid, stearic acid. Most preferably, they are long chain type fatty acids having not less than 12 carbon atoms in a molecule, without an unsaturated bond in a molecule such as laulic acid, myristic acid, palmitic acid and stearic acid.

(C) An inorganic fine particle

A water absorbing agent of the present invention may be added an inorganic fine particle, in particular, an inorganic fine particle of non-dissolving in water to maintain the fluidity after moisture absorption. Specific inorganic powders to be used in the present invention include, for example, metal oxides such as silicon dioxide, titanium oxide, etc.; silica acid (silicate) such as natural zeolite, synthetic zeolite, etc.; kaolin, talc, clay, bentonite, etc. They may be used alone or in combination of two or more kinds. Among these, silicon dioxide and silica acid (silicate salt) are more preferable and silicon dioxide and silica acid (silicate salt) with average particle diameter, measured by a coulter counter method, of 0.001 to 200 $\mu$m are further preferable.

(D) A composite hydrated oxide

A water absorbing agent of the present invention may further be added a composite hydrated oxide containing zinc and silicon or zinc and aluminum to add superior fluidity after moisture absorption (powder fluidity after the water-absorbing resin or the water absorbing agent absorbs moisture) and further superior deodorization performance.

(E) Others

Other additives such as a antimicrobial, an aqueous polymer, water, an organic fine particle, and the like may be added arbitrarily, as long as the water absorbing agent of the present invention can be obtained.

(15) A particulate water absorbing agent of the present invention

A particulate water absorbing agent of the present invention, obtained by the production methods 1 to 3, as an example, is a novel water absorbing agent exhibiting novel performance not conventionally available.

That is, the first particulate water absorbing agent of the present invention is:

a particulate water absorbing agent comprising a water-absorbing resin obtained by crosslinking polymerization of an unsaturated monomer containing an acid group and/or a salt thereof as a main component, and the water absorbing agent satisfies the following (a) to (d):

(a) centrifuge retention capacity (CRC) in a physiological saline solution being in the range of not lower than 32 g/g;

(b) mass median particle size (D50) being in the range of 200 to 400 μm;

(c) ratio of particles having diameter of smaller than 150 μm being in the range of 0 to 2% by weight; and

(d) increased extractables by deterioration expressed by the mentioned above formula of 0 to 15% by weight and extractables for one hour in deterioration test liquid of 0.1 to 30% by weight.

A second particulate water absorbing agent of the present invention is:

a particulate water absorbing agent comprising a water-absorbing resin obtained by crosslinking polymerization of an unsaturated monomer containing an acid group and/or a salt thereof as a main component, and the water absorbing agent satisfies the following (a) to (c) and (e) :

(a) centrifuge retention capacity (CRC) in a physiological saline solution being in the range of not lower than 32 g/g;

(b) mass median particle size (D50) being in the range of 200 to 400 μm;

(c) ratio of particles having diameter of smaller than 150 μm being in the range of 0 to 2% by weight; and

(e) increased ratio of extractables by deterioration expressed by the mentioned above formula of 1 to 4 times, and extractables for one hour in deterioration test liquid of 0.1 to 30% by weight.

A third particulate water absorbing agent of the present invention is:

a particulate water absorbing agent comprising a water-absorbing resin obtained by crosslinking polymerization of an unsaturated monomer containing an acid group and/or a salt thereof as a main component, and the water absorbing agent satisfies the following (a) to (c), (f) and (g) :

(a) centrifuge retention capacity (CRC) in a physiological saline solution being in the range of not lower than 32 g/g;

(b) mass median particle size (D50) being in the range of 200 to 400 μm;

(c) ratio of particles having diameter of smaller than 150 μm being in the range of 0 to 2% by weight;

(f) extractables for 16 hours in a physiological saline solution being in the range of 0.1 to 10% by weight; and

(g) absorbency against pressure at 4.8 kPa (AAP4.8 kPa) in a physiological saline solution being not lower than 21 g/g.

A water absorbing agent of the present invention is controlled to have (b) mass median particle size (D50) of usually as narrow range as 180 to 400 μm, preferably 200 to 400 μm, more preferably 225 to 380 μm and particularly preferably 250 to 350 μm and (c) ratio of particles with diameter of smaller than 150 μm to be 0 to 3% by weight, preferably 0 to 2% by weight and more preferably 0 to 1% by weight. Particle size adjustment is preferably carried out before surface crosslinking, however, it may be adjusted by pulverizing, classification and agglomerating after surface crosslinking. When particle size distribution is out of this range, suitable absorption characteristics and high property can not be obtained in a diaper application. In particular, the item (b) affects absorption characteristics of the water absorbing agent in absorbing articles such as a diaper and when mass median particle size is over 400 μm, surface area per unit weight is decreased, which reduces contact area between the water absorbing agent and urine, and the like, therefore longer absorption period is required to absorb aqueous liquid. On the other hand, when mass median particle size is smaller than 180 μm, surface area per unit weight is increased, which shortens absorption period, however, penetration speed of deteriorated components by urine into particle inside increase simultaneously, which may accelerates deterioration speed by urea. Increase in fine particles also requires regeneration and is not preferable in view of cost. Furthermore, in continuous production, fine particles with diameter of smaller than 150 μm, which is out of treatment capacity of classifier, may generate and make control difficult of ratio of particles with diameter of smaller than 150 μm.

When (c) ratio of particles with diameter of smaller than 150 μm is over 3% by weight, it may lower absorption ability due to gel blocking in absorbing articles, or swollen gel, by absorbing body fluid in practical application, may directly contact with skin (gel on skin) of a user, due to fall off of fine particulate powders before swelling, from inside of absorbing articles to a surface top sheet. Furthermore, loss by scattering in production of absorbing articles or bad effects to work environment may happen and thus not preferable.

The water absorbing agent of the present invention has the above particle size distribution and (a) centrifuge retention capacity (CRC) in a physiological saline solution of not smaller than 32 g/g, preferably 33 to 75 g/g, more preferably 35 to 70 g/g, further preferably 40 to 65 g/g and particularly preferably 45 to 60 g/g. When centrifuge retention capacity (CRC) is smaller than 32 g/g, it requires high quantity of the water absorbing agent to secure desired absorption capacity of absorbing articles and thus practically not preferable.

A water absorbing agent of the present invention satisfies the above characteristics and (d) increased extractables by deterioration of generally 0 to 15% by weight, more preferably 0 to 12% by weight, further preferably 0 to 10% by weight, particularly preferably 0 to 8% by weight and most preferably 0 to 5% by weight. Reason for using a physiological saline solution containing 0.05% by weight of L-ascorbic acid, as deterioration test liquid in measurement of "increased extractables by deterioration" is that deterioration of the water absorbing agent by urine is caused by L-ascorbic acid contained in urine, and that the osmotic pressure is matched to that of body fluid. Increased extractables by deterioration, as shown by the formula, is comparative absolute amount of extractables increased in deterioration by urine, therefore the smaller value means less deterioration. In the present invention, increased extractables by deterioration in the range of 0 to 15% by weight shows stability of the water absorbing agent against body fluid such as urine and thus the present invention has features in the value range itself. When increased extractables by deterioration is over 15% by weight, stability of the water absorbing agent against body fluid such as urine is insufficient and sufficient absorption ability cannot be fulfilled in long period practical application of absorbing substrate.

Similarly, (e) increased ratio of extractables by deterioration of the present invention is usually 1 to 4 times, preferably 1 to 3 times, more preferably 1 to 2 times, further preferably 1 to 1.5 times and particularly preferably 1 to 1.3 times. Increased ratio of extractables by deterioration represents generation ratio of extractables by deterioration and thus the smaller value means less deterioration by urine. Increased ratio of extractables by deterioration in the range of 1 to 4 times shows stability of the water absorbing agent against body fluid such as urine and when increased ratio of extractables by deterioration is over 4 times, stability of the water absorbing agent against body fluid such as urine is insufficient and sufficient absorption ability cannot be fulfilled in long period practical application of absorbing substrate.

Extractables for 1 hour in deterioration test liquid is preferably not more than 0.1 to 30% by weight, more preferably not more than 0.2 to 25% by weight, further preferably not more than 0.3 to 22% by weight, particularly preferably not more than 0.4 to 20% by weight and most preferably not more than 0.5 to 18% by weight. When extractables for 1 hour in deterioration test liquid is over the range, swollen gel in long period use deteriorates with time and extractables increases. The extractables is exuded from an absorbing substrate and may inhibit liquid diffusion of blood or urine into the absorbing substrate. Attainment of the level lower than the lower limit is not restricted, however, it is specified in consideration of production conditions such as cost.

When the item (d) increased extractables by deterioration and/or (e) increased ratio of extractables by deterioration are over the upper limit range, maintaining becomes difficult of body fluid such as urine absorbed by swollen gel, due to significant destruction of crosslinked structure of the water absorbing agent, which may incur increased rewet amount in application such as a diaper. Moreover, swollen gel with destructed crosslinked structure is converted to an aqueous polymer, which may cause leak-out of a fluidized polymer to surface of absorbing articles such as a diaper. Therefore, it may incur increased uncomfortable feeling to persons fitting absorbing articles and is not preferable.

As described above, particle size distribution correlates to increased extractables by deterioration and increased ratio of extractables by deterioration, therefore, the water absorbing agent with (b) mass median particle size D50 adjusted to relatively small level of not larger than 400 μm, along with inhibited (d) increased extractables by deterioration and (e) increased ratio of extractables by deterioration has not been present up to now. However, in the present invention, by introducing (d) increased extractables by deterioration and (e) increased ratio of extractables by deterioration and controlling each to be 0 to 15% by weight and 1 to 4 times, the water absorbing agent superior in use feeling and long period absorption properties can be obtained. Such the water absorbing agent can be produced by the above described methods, however, as shown by Examples, it can be produced by methods other than those described above.

In the present invention, it may be enough that either of (d) increased extractables by deterioration or (e) increased ratio of extractables by deterioration is satisfied, however, it is preferable that both are satisfied simultaneously. In the present invention, as shown later in a measurement method in Example, swollen gel obtained by the addition of 1 g of the water absorbing agent in 25 ml of deterioration test liquid is used based on consideration of 25 times swelling of a diaper and it was found that increased extractables by deterioration or increased ratio of extractables by deterioration correlates to practical use of a diaper and that a water absorbing agent with specific particle size distribution, specific absorption capacity and (d) increased extractables by deterioration or (e) increased ratio of extractables by deterioration provides a high property diaper, irrespective of change in urine composition or use period also in practical application. Evaluation of extractables by dispersing a water-absorbing resin in far excess amount of a physiological saline solution at room temperature or property thereof described in JP-A-8-337726, and the like, has no correlation to practical use and thus meaningless.

Extractables for 1 hour in a physiological saline solution of a water absorbing agent of the present invention is essentially not higher than 50% by weight, preferably 0.1 to 30% by weight, more preferably 0.2 to 25% by weight, further preferably 0.3 to 20% by weight, further more preferably 0.4 to 15% by weight, particularly preferably 0.5 to 10% by weight and most preferably 0.5 to 8% by weight. When extractables for 1 hour is over the upper limit range, extractables is exuded to absorbing substrate in water absorption, which may inhibit liquid diffusion of such as blood and urine to absorbing substrate. Attainment of the level less than 0.1% by weight is generally difficult and does not counterbalance with cost.

In the case of a particulate water absorbing agent with arbitrary level of (d) increased extractables by deterioration or (e) increased ratio of extractables by deterioration, that is in the case of the third particulate water absorbing agent, (f) extractables for 16 hours in a physiological saline solution is 0.1 to 10% by weight, preferably 0.6 to 8% by weight and particularly 0.7 to 5% by weight. When it is over 10% by weight, extractables is exuded from the absorbing substrate in water absorption, which may inhibit liquid diffusion of such as blood and urine to the absorbing substrate. Moreover when deterioration by urine proceeds, it may be to such proceeding degree as not to maintain crosslinked structure and thus disadvantageous. Furthermore, (g) Absorbency Against Pressure at 4. 8 kPa (AAP 4.8 kPa) in a physiological saline solution at 4.8 kPa (about 50 g/cm$^2$, about 0.7 psi) is preferably not lower than 21 g/g, more preferably not lower than 22 g/g, further preferably not lower than 23 g/g, particularly preferably not lower than 24 g/g and most preferably not lower than 25 g/g. When that value is less than 21 g/g, liquid contained in a water absorbing agent may leak out of a water absorbing agent by pressure in practical use. It was found out by detailed study on absorption properties that, even if (d) increased extractables by deterioration or (e) increased ratio of extractables by deterioration may not be satisfied, if the requirements (a), (b) and (c), along with " (f) extractables for 16 hours" and "(g) Absorbency Against Pressure at 4.8 kPa (AAP4.8kPa)" are simultaneously satisfied, performance of the absorbing substrate is not lowered even in long period absorption state of urine in practical use. Reason for that is, performance of absorbing substrate can be maintained in practical use, when extractables for 16 hours of a water absorbing agent before destruction is inhibited low, even if crosslinked structure is destructed in certain level by deterioration caused by urine.

The absorbency against pressure at 4.8 kPa (AAP 4.8 kPa) was found in increasing tendency with higher amount of an internal crosslinking agent, when absorption capacity is the same. It is not clear in detail, however, by consideration of the fact that increase in the amount of an internal crosslinking agent lowers deterioration degree by urine, the absorbency against pressure at 4.8 kPa (AAP4.8kPa) seems to have indirect correlation. That is, to get desired performance of a water absorbing agent after deterioration by urine in the absorbing substrate based on estimation of deterioration by urine, performance of a water absorbing agent before deterioration is required to be set in the above range. The upper limit of (g) is not especially restricted, however, about 40 g/g may be sufficient in certain cases in view of cost increase due to difficulty in production.

Even in the third particulate water absorbing agent, it is preferable to further satisfy (d) increased extractables by deterioration or (e) increased ratio of extractables by deterioration.

(16) Other properties of a particulate water absorbing agent of the present invention

(i) absorbency against pressure at 1.9 kPa (AAP1.9kPa) in a physiological saline solution under 1.9 kPa

The absorbency against pressure of a water absorbing agent of the present invention in a physiological saline solution under load of 1.9 kPa pressure (under load) is preferably not lower than 20 g/g, more preferably not lower than 25 g/g, further preferably not lower than 30 g/g and particularly preferably not lower than 35 g/g. When that value is less than 20 g/g, effect of the present invention may not be fulfilled. The upper limit is not especially limited, however, about 60 g/g may be sufficient in certain cases in view of cost increase due to difficulty in production.

(h) Particles with diameter of 150 to 600 μm; (1) Logarithmic standard deviation

Bulk density (specified by JIS K-3362) of a water absorbing agent of the present invention is adjusted to be preferably in the range of 0.40 to 0.90 g/ml and more preferably 0.50 to 0.80 g/ml. Ratio of "(h) particles with diameter between 150 and 600 μm" is preferably 90 to 100% by weight based on total particles, more preferably 95 to 100% by weight and further preferably 98 to 100% by weight. (L) Logarithmic standard deviation (σζ) of particle size distribution is preferably in the range of 0.20 to 0.40, more preferably 0.20 to 0.38 and particularly preferably 0.20 to 0.36. When particles in this range are used in a diaper, superior property can be obtained.

(k) Fluidity after moisture absorption (Blocking ratio)

A water absorbing agent of the present invention is superior in powder handling characteristics due to having low fluidity after moisture absorption described later in Example. Fluidity after moisture absorption is preferably not higher than 0 to 30% by weight, more preferably 0 to 20% by weight, further preferably 0 to 10% by weight and particularly preferably 0 to 5% by weight. Fluidity after moisture absorption over 30% by weight provides a problem such as difficulty in production of a diaper owing to poor powder fluidity. These fluidity after moisture absorptions can be attained by using the additives.

(j) Absorption speed with vortex method

Absorption speed of the water absorbing agent of the present invention is shorter than 60 seconds, preferably 1 to 55 seconds and more preferably 2 to 50 seconds. A water absorbing agent with absorption speed over 60 seconds may not fulfill sufficient absorption ability when used in an absorbing substrate such as a diaper.

(17) An absorbing article

Applications of the particulate water absorbing agent of the present invention are not especially limited, however, the water absorbing agent is used preferably in an absorbing substrate and an absorbing article.

The absorbing substrate of the present invention is obtained using the particulate water absorbing agent. The absorbing substrate in the present invention means one formed using the particulate water absorbing agent and hydrophilic fibers as main components. Content of the water absorbing agent (core concentration) in the absorbing substrate of the present invention, based on total weight of the water absorbing agent and hydrophilic fibers is preferably 20 to 100% by weight, more preferably 30 to 100% by weight and particularly preferably 40 to 100% by weight.

Further, an absorbing article of the present invention is one equipped with the absorbing substrate of the present invention, a surface sheet with liquid permeability and a back sheet with liquid non-permeability.

A method for production of absorbing articles of the present invention may be, for example, as follows: Preparation of the absorbing substrate (absorbing core) by blending or sandwiching fiber substrates and the water absorbing agent of the present invention, followed by sandwiching the absorbing core between the substrate with liquid permeability (the surface sheet) and the substrate with liquid non-permeability (the back sheet) and mounting of elastic parts, diffusion layers, pressure sensitive adhesive tapes, and the like, if necessary, to fabricate an absorbing article, in particular, a diaper for a child, a diaper for an adult or a sanitary napkin. Such absorbing core has density of 0.06 to 0.50 g/cc and compression molded to have basis weight in the range of 0.01 to 0.20 g/cm$^2$. The fiber substrate to be used is exemplified to be hydrophilic fiber, ground wood pulp, or cotton linter, crosslinked cellulosic fiber, rayon fiber, cotton fiber, wool fiber, acetate fiber, vinylon fiber, etc. Preferably they are used as airlied.

A water absorbing agent of the present invention is one exhibiting superior absorption properties. An absorbing article using this specifically includes, sanitary goods starting from a paper diaper for an adult, whose growth is significant recently, a diaper for a child, a sanitary napkin, so to speak a pad for incontinence, and the like, however, not limited to these. Common idea is that due to improvement of a water absorbing agent of the present invention present in an absorbing article so as to obtain an absorbing article with less rewet and significant dry feeling, it can significantly reduce loads of person wearing such goods and nursing staffs.

Examples

The present invention will be elucidated specifically with the following Examples and Comparative Examples, without being limited to the following Examples.

Various performances of a water absorbing agent were measured by the following methods. They were evaluated also by using a water-absorbing resin instead of a water absorbing agent. Electrical equipment was always used under conditions of 100 V and 60 Hz in Examples. A water-absorbing resin, a water absorbing agent and an absorbing article were used under conditions of 25°C±2°C and 50% RH (relative humidity), unless particularly specified. An aqueous solution of 0.90% by weight of sodium chloride was used as a physiological saline solution.

A water-absorbing resin and a diaper on the market and a water-absorbing resin taken out of a diaper which may absorb moisture on distribution, may be used in a comparison test after vacuum drying under reduced pressure (for example, for about 16 hours at 60 to 80°C), as appropriate, to equilibrium moisture content (2 to 8% by weight, about 5% by weight) of the water-absorbing resin.

(a) Centrifuge Retention Capacity (CRC) for a physiological saline solution

A water absorbing agent of 0.20 g was uniformly put in a bag (60 mm × 85 mm) made of unwoven fabric and immersed in a physiological saline solution controlled at 25±2°C. The bag containing the water absorbing agent was taken out of the saline solution after 30 minutes and subjected to dewatering for 3 minutes at 250 G (250 × 9.81 m/sec$^2$) using a centrifuge (Model H-122 small size centrifuge made by Kokusan Corporation) and then weighed to get weight W2 (g). Weight W1 (g) of the bag was measured after similar operation without any water absorbing agent. Centrifuge Retention Capacity (g/g) was calculated from weights W1 and W2 according to the following formula.

$$\text{Centrifuge Retention Capacity (g/g)}$$
$$= ((\text{weight W2 (g)} - \text{weight W1 (g)})/\text{weight of water absorbing}$$
$$\text{agent (g))} - 1$$

(b) Absorbency Against Pressure at 4.8 kPa (AAP4. 8 kPa) in a physiological saline solution

A water absorbing agent of 0.900 g was uniformly scattered on a 400-Mesh wire mesh made of stainless steel (mesh size: 38 $\mu$m) welded to the bottom end face of a plastic support cylinder with inner diameter of 60 mm. A piston (cover plate), which has outer diameter a little smaller than 60 mm, no gap against the inner surface of the support cylinder and can move up and down smoothly, was mounted on the water absorbing agent. Total weight W3 (g) of the support cylinder, the water absorbing agent and the piston was measured. A load adjusted weight to press the water absorbing agent uniformly at 4.8 kPa (about 50/cm$^2$, about 0.7 psi) including the weight of the piston was mounted on the piston, thereby completing a set of measuring apparatus. A glass filter with diameter of 90 mm and thickness of 5 mm was placed in a Petri dish with diameter of 150 mm and a physiological saline solution controlled at 25$\pm$2°C was poured up to the same level as the upper surface of the glass filter. A sheet of filter paper with diameter of 9 cm (No.2 from Toyo Roshi Kaisha Ltd.) was placed on the surface of the glass filter to be entirely wetted, and then excess solution over the wetted filter paper was removed.

The set of the measuring apparatus was placed on the wetted filter paper and the liquid was absorbed with the water absorbing agent under load. The liquid level was kept constant by adding the liquid when the liquid surface became lower than the upper surface of the filter paper. The set of the measuring apparatus was lifted up after an hour and weight W4 (g) (the total weight of the support cylinder, the swollen water absorbing agent and the piston) excluding the load was measuredagain. Theabsorbencyagainstpressure (AAP4.8kPa) (g/g) was calculated from weights W3 and W4 according to the following formula.

$$\text{Absorbency Against Pressure (g/g)}$$
$$= (\text{weight W4 (g)} - \text{weight W3 (g)}) /\text{weight of a water absorbing}$$
$$\text{agent (g)}$$

(c) Absorbency against pressure at 1.9 kPa (AAP1.9 kPa) in a physiological saline solution

The absorbency against pressure (AAP1.9 kPa) was obtained by the same operation and calculation as in the above (b) except that the water absorbing agent was uniformly pressed at 1.9 kPa (about 20 g/cm$^2$, about 0.3 psi), including the weight of the piston.

(d) Mass median particle size (D50), logarithmic standard deviation (5$\zeta$) and percentage by weight of particles less than 150 $\mu$m in diameter.

A water absorbing agent was subjected to sieve classification using JIS standard sieves having mesh opening size of 850 $\mu$m, 710 $\mu$m, 600 $\mu$m, 500 $\mu$m, 425 $\mu$m, 300 $\mu$m, 212 $\mu$m, 150 $\mu$m, 106 $\mu$m and 45 $\mu$m, and percentage by weight of particles less than 150 $\mu$m in diameter was measured, while oversize percentages R at each particle size were plotted on a logarithmic probability paper. Particle size corresponding to R = 50% by weight was thus determined as mass median particle size (D50). Logarithmic standard deviation ($\delta\zeta$) is represented by the following formula, wherein smaller value of $\delta\zeta$ means narrower particle-size distribution.

$$\delta\zeta = 0.5 \times \ln (X2/X1)$$

(wherein X1 and X2 are particle diameters for R = 84.1% by weight and R = 15.9% by weight, respectively)
For sieve classification, a water absorbing agent of 10.00 g is charged into each of the JIS standard mesh sieves (The IIDA TESTING SIEVE: inner diameter of 80 mm) and sieved for 5 minutes using a Ro-tap type sieve shaker (Model ES-65 sieve shaker from Iida Seisakusho Co., Ltd.).
Mass median particle size (D50) means particle diameter for a standard sieve, corresponding to 50% by weight based on the whole particles, when sieving is carried out by standard sieves with particular meshes as described in U.S.

No.5,051,259 etc.

(e) Extractables (extractabless in a physiological saline solution for one hour and 16 hours)

First of all, preparation for measurement of extractabless in a physiological saline solution for one hour and 16 hours (hereinafter, referred to as extractables for one hour and extractables for 16 hours, respectively) will be described. A pH electrode was calibrated with buffer solutions of pH 4.0, pH7. 0 and pH10.0. A physiological saline solution of 50 ml, prepared beforehand for a blank test, was poured into a 100 ml glass beaker and added in dropwise with a 0.1 mol/L aqueous solution of sodium hydroxide until pH 10, while stirring with a stirrer chip with length of 30 mm, to determine titration amount $V_{ab}$ (ml) of the aqueous solution of sodium hydroxide for a blank test. The saline solution was further added in dropwise with a 0.1mol/L aqueous solution of hydrochloric acid until pH 2. 7, while stirring, to determine titration amount $V_{bb}$ (ml) of hydrochloric acid for a blank test.

A physiological saline solution of 200 ml, prepared beforehand, was poured into a 250 ml polypropylene cup with a lid and added with 1.0 g (= m (g)) of a water absorbing agent obtained in Examples or Comparative Examples to be described later. The saline solution was stirred with a stirrer with length of 30 mm and diameter of 8 mm at 500 $\pm$ 50 rpm for one hour or 16 hours to extract extractables. After stirring for one hour or 16 hours, the saline solution was filtered with a filter paper (No. 2, made by Toyo Roshi Kaisha Ltd. , retaining particle size of 5 $\mu$m according to JIS P 3801), to obtain a filtrate.

Thus obtained filtrate of 20 ml (recorded as F (ml)) was poured into a 100 ml glass beaker and added with a physiological saline solution to obtain 50 ml of a filtrate for titration. When thus obtained filtrate is less than 20 ml, the total amount of thus obtained filtrate was recorded as F(ml) and added with a 0.90% by weight aqueous solution of sodium chloride to obtain 50 ml of a filtrate for titration.

Subsequently, the filtrate for titration was added in dropwise with a 0.1mol/L aqueous solution of sodium hydroxide, while stirring with a cylinder-type stirrer with length of 30 mm and diameter of 8 mm until pH 10, to determine titration amount $V_a$ (ml) of the aqueous solution of sodium hydroxide. The filtrate was further added in dropwise with a 0.1 mol/L aqueous solution of hydrochloric acid, while stirring, until pH 2.7, to determine titration amount $V_b$ (ml) of the aqueous solution of hydrochloric acid. Extractables (%) is calculated by the following formula.

$$\text{Extractables } (\%) = ((W_a + W_b) / m) \times 100$$

Wherein $W_a$ (g) is relative weight of units having an acid group of extractables in a water absorbing agent and $W_b$ (g) is relative weight of units having a carboxylate group neutralized by an alkali metal of extractables in a water absorbing agent. Each of them is calculated by the following formulas.

$$W_a \text{ (g)} = N_a \times 72 \times 200/F$$

$$W_b \text{ (g)} = N_b \times 94 \times 200/F$$

Wherein the value 72 is weight per 1 mole of a repetition unit in polyacrylic acid, and when a monomer with an acid group other than acrylic acid is copolymerized, the value is altered to average weight of a repetition unit including suchamonomer. The value 94 is weight per 1 mole of a repetition unit in polysodium acrylate and it is altered, as appropriate, when a monomer having an acid group other than acrylic acid is copolymerized, or when potassium, lithium, and the like is used instead of sodium as an alkali metal salt.

$N_a$ (mol) is number of moles of an acid group of extractables in a filtrate and $N_b$ (mol) is number of moles of a carboxylate group neutralized by an alkali metal, of extractables in a filtrate. Each of them is calculated by the following formulas.

$$N_a \text{ (mol)} = (V_a - V_{ab})/1000 \times 0.1$$

$$N_b \text{ (mol)} = N_1 - N_a$$

Wherein $N_1$ (mol) is number of total moles of extractables in a filtrate to be measured and is calculated by the following formula.

$$N_1 \ (mol) \ = \ (V_b \ - \ V_{bb})/1000 \ \times \ 0.1$$

The extractabless calculated by the formulas were differentiated as extractables for one hour (%) on a filtrate obtained by stirring a physiological saline solution for one hour, and extractables for 16 hours (%) on a filtrate obtained by stirring a physiological saline solution for 16 hours.

(f) Evaluation of resistance to urine (extractables for one hour in deterioration test liquid, increased extractables by deterioration, increased ratio of extractables by deterioration)

A physiological saline solution prepared beforehand was added with L-ascorbic acid so as to obtain deterioration test liquid of 0.05% by weight. Specifically, 0.50 g of L-ascorbic acid was dissolved in 999. 5 g of a physiological saline solution to prepare deterioration test liquid.

Deterioration test liquid of 25 ml was poured into a 250 ml polypropylene cup with a lid and added with 1.0 g of a water absorbing agent to form swollen gel. After the cup was sealed with the lid, the swollen gel was left for standing in atmosphere of 37°C for 16 hours.

After 16 hours, 175 ml of the physiological saline solution was added and then extractables after deterioration was extracted from the hydrated gel by stirring for one hour similarly as in (d) with a cylinder-type stirrer with length of 30 mm and diameter of 8 mm.

After extraction by stirring for one hour, the saline solution was filtered and pH-titrated by the same method as in the previous item (d) to determine extractables for one hour (%) in deterioration test liquid using the same formulas as in (d). To compare absolute amount of increased extractables by deterioration, for evaluation of resistance to urine, increased extractables by deterioration (% by weight) was calculated by the following formula.

$$\text{Increased extractables by deterioration (\% by weight)}$$
$$= \text{extractables for one hour (\% by weight) in deterioration}$$
$$\text{test liquid} - \text{extractables for one hour (\% by weight) in a}$$
$$\text{physiological saline solution}$$

To compare extractables formed by deterioration with extractabless before deterioration, for evaluating resistance to urine, increased ratio of extractables by deterioration (quotient) was calculated by the following formula.

$$\text{Increased ratio of extractables by deterioration}$$
$$= \text{extractables for one hour (\% by weight) in deterioration}$$
$$\text{test liquid/extractables for one hour (\% by weight) in a}$$
$$\text{physiological saline solution}$$

(g) Evaluation of absorption speed (Vortex method)

A 0.90% by weight aqueous solution of sodium chloride (physiological saline solution) of 1,000 parts by weight was added with 0.02 part by weight of a brilliant blue-FCF, a food additive, and kept at 30°C. The physiological saline solution of 50 ml was poured into a 100 ml beaker and added with 2. 0 g of a water absorbing agent, while stirring at 600 rpm with a cylinder-type stirrer with length of 40 mm and diameter of 8 mm, to measure absorption speed (second). Absorption speed (second) is time required for the test liquid to completely cover the stirrer chip as the water absorbing agent absorbs the physiological saline solution, which was measured according to the standard described in JIS K 7224 (1996) "Testing method for water absorption speed of super absorbent resins - Description"

(h) Fluidity after moisture absorption (% by weight)

A water absorbing agent of 2 g was uniformly scattered on the bottom of an aluminum cup with diameter of 52 mm and height of 22 mm and quickly put in a humidity-controllable incubator (PLATIOOUS LUCIFER PL-2G from ESPEC Corp.) controlled beforehand at 25°C and 90% relative humidity, and left for standing for 60 minutes. The moisture-absorbed water absorbing agent was transferred to a JIS standard sieve with diameter of 7.5 cm and 2,000 μm of mesh open. when the moisture-absorbed water absorbing agent was adhered to aluminum cup too rigidly to do so, the water absorbing agent should be torn off the cup and transferred to the sieve very carefully not to destroy the block. The transferred water absorbing agent to the sieve was immediately sieved for 8 seconds using a sieve shaker (IIDA SIEVE SHAKER, TYPE: ES-65, SER.No.0501). Weight W5 (g) of the oversize water absorbing agent left on the sieve and weight W6 (g) of the undersize water absorbing agent passed through the sieve were measured. Fluidity after moisture absorption (Blocking ratio) (% by weight) was calculated by the following formula. The lower fluidity after moisture absorption means, the better water absorbing agent in fluidity after moisture absorption and in powder handling nature.

$$\texttt{Fluidity after moisture absorption (\% by weight)}$$
$$\texttt{= (weight W5 (g)/(weight W5 (g) + weight W6 (g)))} \times 100$$

(i) Deodorization test (evaluation of a water absorbing agent)

Urine of 50 m, collected from 20 adults, was poured into a 120 ml polypropylene cup with a lid and added with 2.0 g of a water absorbing agent to form swollen gel. The urine used was one within 2 hours after excretion. The cup was capped and the swollen gel was kept at 37°C. The lid was removed after 6 hours from start of liquid absorption and 20 adult panelists evaluated deodorization effect by smelling the contents at about 3 cm above the top of the cup. Each panelist recorded scores according to the 6 grades of the following evaluation criterion to determine an average score. Urine without a water absorbing agent was evaluated similarly and graded as score 5 to be used as evaluation standard.

0: no odor
1: barely perceivable odor
2: perceivable but allowable odor
3: easily perceivable odor
4: strong odor
5: very strong odor

(j) Performance evaluation as an absorbing substrate (Rewet amounts after 10 minutes and by deterioration)

An absorbing substrate for evaluation was prepared to evaluate a water absorbing agent as an absorbing substrate and subjected to a rewet test.
To begin with, a method for preparation of an absorbing substrate for evaluation is shown below.
A water absorbing agent to be described later of 1 part by weight and crushed wood pulp of 2 parts by weight were subj ected to dry mixing using a mixer. Thus obtained mixture was spread on a wire screen of 400-Mesh (mesh size: 38 μm) to form a web with diameter of 90 mm. The web was pressed under pressure of 196.14 kPa (2 kgf/cm$^2$) for 1 minute to obtain an absorbing substrate for evaluation with basis weight of 0.05 g/cm$^2$.
Subsequently, a method for evaluation of rewet amount after 10 minutes is shown below.
The absorbing substrate for evaluation was placed on the bottom of a Petri dish with inner diameter of 90 mm made of stainless steel and nonwoven fabric with diameter of 90 mm was placed thereon. Deterioration test liquid of 30 ml used in the item (f) ; evaluation of resistance to urine, was then poured over the nonwoven fabric and subjected to absorption for 10 minutes under conditions of no load. Subsequently, 30 sheets of filter paper with diameter of 90mm (No. 2 from Toyo Roshi Kaisha Ltd.), whose weight (W7(g)) was measured beforehand, was placed on the nonwoven and the absorbing substrate. And then, a piston and a load (total weight of the piston and load was 20kg) with diameter of 90mm were placed on the filter paper so as to press uniformly the absorbing substrate, the nonwoven and the filter paper. The filter papers were made to absorb rewet liquid, while pressing for 5 minutes. The 30 sheets of the filter papers were then weighed (W8 (g)) to calculate rewet amount after 10 minutes.

$$\texttt{Rewet amount after 10 minutes (g) = W8 (g) - W7 (g)}$$

A method for evaluation of rewet amount by deterioration is shown below.

Deterioration test liquid of 30 ml, prepared beforehand by processing similarly as above, was poured over the nonwoven fabric and an absorbing substrate for evaluation, and was absorbed under condition of no load and left for standing in atmosphere of 37°C for 16 hours. The Petri dish was put in a sealed polyethylene bag with size of 140 mm × 200 mm to prevent moisture from evaporating during standing.

After specified time, subsequently, 30 sheets of filter paper with diameter of 90mm (No. 2 from Toyo Roshi Kaisha Ltd.), whose weight (W9(g)) was measured beforehand, was placed on the nonwoven and the absorbing substrate. And then, a piston and a load (total weight of the piston and load was 20kg) with diameter of 90mm were placed on the filter paper so as to press uniformly the absorbing substrate, the nonwoven and the filter paper. The filter papers were made to absorb rewet liquid, while pressing for 5 minutes. The 30 sheets of the filter papers (W10 (g)) were then weighed to calculate rewet amount by deterioration.

$$\text{Rewet amount by deterioration (g) = W10 (g) - W9 (g)}$$

(Reference Example 1)

Polyethylene glycol diacrylate (average added mole number of ethylene oxide unit: 9) of 4.3 g was dissolved in 1,500 g of an aqueous solution of sodium acrylate having neutralization ratio of 75% by mole (monomer concentration: 24% by weight) to make reaction liquid. Thus obtained reaction liquid was poured into a tray with length of 320 mm, width of 220 mm and height of 50 mm made of stainless steel, with the reaction liquid being 17 mm deep. The stainless steel tray was immersed in a water bath at 30°C, after the top of the tray was sealed with a polyethylene film having a nitrogen gas inlet, an exhaust gas outlet and an inlet of a polymerization initiator. Nitrogen gas was introduced into the reaction liquid to purge dissolved oxygen in the liquid, while controlling the reaction liquid temperature at 30°C. Subsequently, nitrogen gas continued to be introduced in an upper space of the reactor, while exhausting from the other side. The reaction liquid was added with, as polymerization initiators, 5.1 g of a 10% by weight aqueous solution of 2,2'-azobis(2-amidinopropane)dihydrochloride, 2.5 g of a 10% by weight aqueous solution of sodium persulfate, 0.4 g of a 1% by weight aqueous solution of L-ascorbic acid and 2.2 g of a 0.35% by weight aqueous solution of hydrogen peroxide, and stirred sufficiently with a magnetic stirrer. As polymerization began in 1 minute after the addition of the polymerization initiators, the stainless steel tray was intermittently immersed in a water bath at 12°C by 10 mm in height from the tray bottom to control polymerization temperature. As peak temperature of polymerization of 80°C was attained after 55 minutes from initiation of polymerization, the tray was immersed in a water bath at 70°C up to 10 mm in height from the tray bottom to mature gel and left for 60 minutes. Thus obtained hydrated gel-like polymer was crushed by a meat chopper (Model No. 32 meat chopper from Hiraga Seisakusho Inc.) equipped with a die having bore diameter of 9.5 mm, spread on a wire net of 50-Mesh (mesh size: 300 $\mu$m) and dried with hot air at 160°C for 60 minutes. Thus dried material was then pulverized by a roll mill and sieved with wire meshes with mesh opening sizes of 710 $\mu$m and 150 $\mu$m to obtain irregularly pulverized powder of a water-absorbing resin (a). Centrifuge Retention Capacity (CRC), mass median particle size (D50), and percentage by weight of particles less than 150 $\mu$m in diameter of thus obtained water-absorbing resin (a) are shown in Table 1. Water-absorbing resins (b) to (1) obtained in the following Reference Examples were also evaluated similarly and results of the evaluation are shown in Table 1.

Thus obtained powder of a water-absorbing resin (a) of 100 parts by weight was mixed with 3.9 parts by weight of a surface crosslinking agent composed of 0.55 part by weight of propylene glycol, 0.35 part by weight of 1,4-butanediol and 3 parts by weight of water. The mixture was heated at heating medium temperature of 210°C for 40 minutes to obtain a water-absorbing resin (1).

(Reference Example 2)

Polyethylene glycol diacrylate (average added mole number of ethylene oxide unit: 9) of 4.0 g was dissolved in 5,500 g of an aqueous solution of sodium acrylate having neutralization ratio of 75% by mole (monomer concentration: 40% by weight) to make reaction liquid. Subsequently, a reactor fabricated by attaching a lid to a 10-L twin-arm type kneader made of stainless steel and equipped with a jacket and two $\Sigma$-shaped agitating blades was supplied the reaction liquid and the reaction system was purged dissolved oxygen by introducing with nitrogen gas, while keeping the reaction liquid at 30°C. The reaction liquid was then added with 29. 8 g of a 10% by weight aqueous solution of sodium persulfate and 1.5 g of a 1% by weight aqueous solution of L-ascorbic acid, while stirring the reaction liquid, resulting in initiation of polymerization after 1 minute. Peak temperature of polymerization of 93°C was attained after 15 minutes from initiation of polymerization. After 60 minutes from initiation of polymerization, a hydrated gel-like polymer was taken out, which was in crushed state to particles with diameter of 1 to 4 mm. The crushed, hydrated gel-like polymer was spread on a wire net of 50-Mesh (mesh size: 300 $\mu$m) and dried with hot air at 160°C for 60 minutes. Thus dried material was then pulverized by a roll mill and sieved with wire meshes with mesh opening sizes of 710 $\mu$m and 150 $\mu$m to obtain irregularly pulverized powder of a water-absorbing resin (b).

Thus obtained powder of a water-absorbing resin (b) of 100 parts by weight was mixed with 3.53 parts by weight of a surface crosslinking agent composed of 0.5 part by weight of propylene glycol, 0.03 part by weight of ethylene glycol diglycidyl ether, 0.3 part by weight of 1,4-butanediol and 2.7 parts by weight of water. The mixture was heated at heating medium temperature of 210°C for 45 minutes to obtain a water-absorbing resin (2).

(Reference Example 3)

Polyethylene glycol diacrylate (average added mole number of ethylene oxide unit: 9) of 2.5 g was dissolved in 5,500 g of an aqueous solution of sodium acrylate having neutralization ratio of 75% by mole (monomer concentration: 38% by weight) to make reaction liquid. After purging dissolved oxygen similarly as in Reference Example 2, the reaction liquid was supplied to the reactor of Reference Example 2 and the reaction system was purged with nitrogen gas, while keeping the reaction liquid at 30°C. The reaction liquid was then added with 29.8 g of a 10% by weight aqueous solution of sodium persulfate and 1.5 g of a 1% by weight aqueous solution of L-ascorbic acid, while stirring the reaction liquid, resulting in initiation of polymerization after about 1 minute. Peak temperature of polymerization of 86°C was attained after 17 minutes from initiation of polymerization. After 60 minutes from initiation of polymerization, a hydrated gel-like polymer was taken out, which was in granulated state to particles with diameter of about 1 to 4 mm. The hydrated gel-like polymer was dried and pulverized similarly as in Reference Example 2, and sieved with wire meshes with mesh opening sizes of 710 $\mu$m and 150 $\mu$m to obtain irregularly pulverized powder of a water-absorbing resin (c).

Thus obtained powder of a water-absorbing resin (c) of 100 parts by weight was mixed with 3.53 parts by weight of the surface crosslinking agent having the same composition as in Reference Example 2. The mixture was heated at heating medium temperature of 195°C for 40 minutes to obtain a water-absorbing resin (3).

(Reference Example 4)

Polyethylene glycol diacrylate (average added mole number of ethylene oxide unit: 9) of 19.6 g and disodium phosphite pentahydrate of 36.3 g were dissolved in 5,500 g of an aqueous solution of sodium acrylate having neutralization ratio of 75% by mole (monomer concentration: 33% by weight) to make reaction liquid. After purging dissolved oxygen similarly as in Reference Example 2, the reaction liquid was supplied to the reactor of Reference Example 2 and the reaction system was purged with nitrogen gas, while keeping the reaction liquid at 30°C. The reaction liquid was then added with 20.5 g of a 10% by weight aqueous solution of sodium persulfate and 1.0 g of a 1% by weight aqueous solution of L-ascorbic acid, while stirring the reaction liquid, resulting in initiation of polymerization after about 1 minute. Peak temperature of polymerization of 85°C was attained after 17 minutes from initiation of polymerization. After 60 minutes from initiation of polymerization, a hydrated gel-like polymer was taken out, which was in crushed state to particles with diameter of about 1 to 4 mm. The hydrated gel-like polymer was dried and pulverized similarly as in Reference Example 2, and sieved with wire meshes with mesh opening sizes of 600 $\mu$m and 150 $\mu$m to obtain irregularly pulverized powder of a water-absorbing resin (d).

Thus obtained powder of a water-absorbing resin (d) of 100 parts by weight was mixed with 3.53 parts by weight of the surface crosslinking agent of the same composition as in Reference Example 2. The mixture was heated at heating medium temperature of 210°C for 35 minutes to obtain a water-absorbing resin (4).

(Reference Example 5)

Polyethylene glycol diacrylate (average added mole number of ethylene oxide unit: 9) of 20.0 g and sodium phosphinate of 3.3 g were dissolved in 5, 500 g of an aqueous solution of sodium acrylate having neutralization ratio of 70% by mole (monomer concentration: 30% by weight) to make reaction liquid. After purging dissolved oxygen similarly as in Reference Example 2, the reaction liquid was supplied to the reactor of Reference Example 2 and the reaction system was purged with nitrogen gas, while keeping the reaction liquid at 30°C. The reaction liquid was then added with 22.6 g of a 10% by weight aqueous solution of sodium persulfate and 1.1 g of a 1% by weight aqueous solution of L-ascorbic acid, while stirring the reaction liquid, resulting in initiation of polymerization after about 1 minute. Peak temperature of polymerization of 82°C was attained after 18 minutes from initiation of polymerization. After 40 minutes from initiation of polymerization, a hydrated gel-like polymer was taken out, which was in crushed state to particles with diameter of about 1 to 4 mm. The hydrated gel-like polymer was dried and pulverized similarly as in Reference Example 2, and sieved with wire meshes with mesh opening sizes of 600 $\mu$m and 150 $\mu$m to obtain irregularly pulverized powder of a water-absorbing resin (e).

Thus obtained powder of a water-absorbing resin (e) of 100 parts by weight was mixed with 3.8 parts by weight of a surface crosslinking agent composed of 0.5 part by weight of propylene glycol, 0.3 part by weight of 1,3-propanediol and 3 parts by weight of water. The mixture was heated at heating medium temperature of 195°C for 40 minutes to obtain a water-absorbing resin (5).

(Reference Example 6)

Tetraallyloxyethane of 1.9 g was dissolved in 1,500 g of an aqueous solution of acrylic acid (monomer concentration: 20% by weight) to prepare reaction liquid. Thus obtained reaction liquid was poured into the tray made of stainless steel of Reference Example 1, with the reaction liquid being 17 mm deep. The stainless steel tray was sealed similarly as in Reference Example 1 and immersed in a water bath at 20°C. Nitrogen gas was introduced into the reaction liquid to purge dissolved oxygen in the liquid, while controlling the reaction liquid temperature at 20°C. Subsequently, nitrogen gas continued to be introduced in an upper space of the reactor, while exhausting from the other side. The reaction liquid was then added with, as polymerization initiators, 8.3 g of a 10% by weight aqueous solution of 2,2'-azobis(2-amidinopropane)dihydrochloride, 0.6 g of a 5% by weight aqueous solution of L-ascorbic acid and 2.1 g of a 3.5% by weight aqueous solution of hydrogen peroxide, while stirring the reaction liquid with a magnetic stirrer, resulting in initiation of polymerization after about 1 minute. Cooling and heating of the reactant was repeated from the bottom surface of the tray during the polymerization reaction. Peak temperature of polymerization of 75°C was attained after 35 minutes from initiation of polymerization. After 90 minutes from initiation of polymerization, a hydrated gel-like polymer was taken out. Thus obtained hydrated gel-like polymer was cut with scissors into squares with sides of about 5 cm. Thus obtained hydrated gel-like polymer in squares with sides of about 5 cm was then fed at constant rate to the same meat chopper as in Reference Example 1, while 702 g of a 22% by weight aqueous solution of sodium carbonate was added at constant rate for neutralization. The crushed gel discharged from the meat chopper was left in atmosphere of about 70°C until the gel did not turn red any longer even when phenolphthalein liquid was poured over the gel. The gel was then dried and pulverized similarly as in Reference Example 1 and sieved with wire meshes with mesh opening sizes of 600 $\mu$m and 150 $\mu$m to obtain irregularly pulverized powder of a water-absorbing resin (f).

Thus obtained powder of a water-absorbing resin (f) of 100 parts by weight was mixed with 3.83 parts by weight of a surface crosslinking agent composed of 0.5 part by weight of propylene glycol, 0.03part by weight of ethylene glycol diglycidyl ether, 0.3 part by weight of 1,3-propanediol and 3 parts by weight of water. The mixture was heated at heating medium temperature of 195°C for 40 minutes to obtain a water-absorbing resin (6).

(Reference Example 7)

The dried material of the hydrated gel-like polymer obtained in Reference Example 2 was pulverized by the similar roll mill as in Reference Example 2, setting pulverizing conditions so as to obtain finer particles than in Reference Example 2 and sieved with wire meshes having mesh opening sizes of 425 $\mu$m and 150 $\mu$m to obtain irregularly pulverized powder of a water-absorbing resin (g).

Thus obtained powder of a water-absorbing resin (g) of 100 parts by weight was mixed with 4.93 parts by weight of a surface crosslinking agent composed of 1 part by weight of propylene glycol, 0.03 part by weight of ethylene glycol diglycidyl ether, 3 parts by weight of water and 0.9 part by weight of methanol. The mixture was heated at heating medium temperature of 210°C for 45 minutes to obtain a water-absorbing resin (7).

(Reference Example 8)

The dried material of the hydrated gel-like polymer obtained in Reference Example 3 was pulverized by the similar roll mill as in Reference Example 3, setting pulverizing conditions so as to obtain finer particles than in Reference Example 3 and sieved with wire meshes having mesh opening sizes of 500 $\mu$m and 150 $\mu$m to obtain irregularly pulverized powder of a water-absorbing resin (h).

Thus obtained powder of a water-absorbing resin (h) of 100 parts by weight was mixed with 3.53 parts by weight of the surface crosslinking agent of the same composition as in Reference Example 3. The mixture was heated at heating medium temperature of 210°C for 45 minutes to obtain a water-absorbing resin (8).

(Reference Example 9)

The dried material of the hydrated gel-like polymer obtained in Reference Example 2 was pulverized by the similar roll mill as in Reference Example 2, setting pulverizing conditions so as to obtain coarser particles than in Reference Example 2 and sieved with wire meshes having mesh opening sizes of 850 $\mu$m and 106 $\mu$m to obtain irregularly pulverized powder of a water-absorbing resin (i).

Thus obtained powder of a water-absorbing resin (i) of 100 parts by weight was mixed with 3.53 parts by weight of the similar surface crosslinking agent as in Reference Example 2. The mixture was heated at heating medium temperature of 210°C for 55 minutes to obtain a water-absorbing resin (9).

(Reference Example 10)

The dried material of the hydrated gel-like polymer obtained in Reference Example 3 was pulverized by the similar roll mill as in Reference Example 9, setting pulverizing conditions so as to obtain still coarser particles than in Reference Example 9 and sieved with wire meshes having mesh opening sizes of a 850 $\mu$m and 150 $\mu$m to obtain irregularly pulverized powder of a water-absorbing resin (j).

Thus obtained powder of a water-absorbing resin (j) of 100 parts by weight was mixed with 3.53 parts by weight of the similar surface crosslinking agent as in Reference Example 2. The mixture was heated at heating medium temperature of 195°C for 45 minutes to obtain a water-absorbing resin (10).

(Reference Example 11)

A water-absorbing resin (11) was obtained by processing similarly as in Reference Example 4 except that disodium phosphite pentahydrate was not added as in Reference Example 4.

[Example 1]

The water-absorbing resin (1) obtained in Reference Example 1 was used as it is as a water absorbing agent (1). Evaluation results on centrifuge retention capacity, absorbency against pressure at 4.8 kPa, extractables for 16 hours in a physiological saline solution, absorbency against pressure at 1.9 kPa, urine resistance, absorption speed and particle size distribution of the water absorbing agent (1) are shown in Tables 2 to 4.

[Example 2]

To 100 parts by weight of the water-absorbing resin (2) obtained in Reference Example 2, 2 parts by weight of a sodium diethylenetriamine pentaacetate aqueous solution was mixed by spraying so that content of sodium diethylenetriamine pentaacetate became 50 ppm to the water-absorbing resin (2). Thus obtained mixture was cured at 60°C for 1 hour to obtain a water absorbing agent (2). The water absorbing agent (2) was evaluated in the same manner as in Example 1. Results are shown in Tables 2 to 4. By applying the spray mixing process of the aqueous solution and the curing process, the water absorbing agent (2) obtained was in agglomelated state.

[Example 3]

To 100 parts by weight of the water-absorbing resin (3) obtained in Reference Example 3, 2 parts by weight of sodium diethylenetriamine pentaacetate aqueous solution was mixed by spraying so that content of sodium diethylenetriamine pentaacetate became 100 ppm to the water-absorbing resin (3). Thus obtained mixture was cured at 60°C for 1 hour to obtain a water absorbing agent (3). The water absorbing agent (3) was evaluated in the same manner as in Example 1. Results are shown in Tables 2 to 4. By applying the spray mixing process of the aqueous solution and the curing process, the water absorbing agent (3) obtained was in agglomerated state.

[Example 4]

To 100 parts by weight of the water-absorbing resin (4) obtained in Reference Example 4, 2 parts by weight of water was mixed by spraying. Thus obtained mixture was cured at 60°C for 1 hour to obtain a water absorbing agent (4). The water absorbing agent (4) was evaluated in the same manner as in Example 1. Results are shown in Tables 2 to 4. By applying the water spray mixing process and the curing process, the water absorbing agent (4) obtained was in agglomerated state.

[Example 5]

The same procedures as in Example 4 were repeated except for using the water-absorbing resin (5) obtained in Reference Example 5 instead of the water-absorbing resin (4) obtained in Reference Example 4, to obtain a water absorbing agent (5). The water absorbing agent (5) was evaluated in the same manner as in Example 1. Results are shown in Tables 2 to 4. By applying the water spray mixing process and the curing process, the water absorbing agent (5) obtained was in agglomerated state.

[Example 6]

The same procedures as in Example 4 were repeated except for using the water-absorbing resin (6) obtained in Reference Example 6 instead of the water-absorbing resin (4) obtained in Reference Example 4, to obtain a water absorbing agent (6). The water absorbing agent (6) was evaluated in the same manner as in Example 1. Results are shown in Tables 2 to 4. By applying the water spray mixing process and the curing process, the water absorbing agent (6) obtained was in agglomerated state.

[Example 7]

The same procedures as in Example 2 were repeated except for using the water-absorbing resin (7) obtained in Reference Example 7 instead of the water-absorbing resin (2) obtained in Reference Example 2, to obtain a water absorbing agent (7). The water absorbing agent (7) was evaluated in the same manner as in Example 1. Results are shown in Tables 2 to 4. By applying the water spray mixing process and the curing process, the water absorbing agent (7) obtained was in agglomerated state.

[Example 8]

The same procedures as in Example 3 were repeated except for using the water-absorbing resin (8) obtained in Reference Example 8 instead of the water-absorbing resin (3) obtained in Reference Example 3, to obtain a water absorbing agent (8). The water absorbing agent (8) was evaluated in the same manner as in Example 1. Results are shown in Tables 2 to 4. By applying the water spray mixing process and the curing process, the water absorbing agent (8) obtained was in agglomerated state.

[Comparative Example 1]

The same procedures as in Example 2 were repeated except for using the water-absorbing resin (9) obtained in Reference Example 9 instead of the water-absorbing resin (2) obtained in Reference Example 2, to obtain a comparative water absorbing agent (1). The comparative water absorbing agent (1) was evaluated in the same manner as in Example 1. Results are shown in Tables 2 to 4. By applying the water spray mixing process and the curing process, the comparative water absorbing agent (1) obtained was in agglomerated state.

[Comparative Example 2]

The same procedures as in Example 3 were repeated except for using the water-absorbing resin (10) obtained in Reference Example 10 instead of the water-absorbing resin (3) obtained in Reference Example 3, to obtain a comparative water absorbing agent (2). The comparative water absorbing agent (2) was evaluated in the same manner as in Example 1. Results are shown in Tables 2 to 4. By applying the water spray mixing process and the curing process, the comparative water absorbing agent (2) obtained was in agglomerated state.

[Comparative Example 3]

The same procedures as in Example 2 were repeated except for using water instead of the sodium diethylenetriamine pentaacetate aqueous solution, to obtain a comparative water absorbing agent (3). The comparative water absorbing agent (3) was evaluated in the same manner as in Example 1. Results are shown in Tables 2 to 4. Further, fluidity after moisture absorption was evaluated. Results are shown in Table 5. By applying the water spray mixing process and the curing process, the comparative water absorbing agent (3) obtained was in agglomelated state.

[Comparative Example 4]

The same procedures as in Example 3 were repeated except for using water instead of the sodium diethylenetriamine pentaacetate aqueous solution, to obtain a comparative water absorbing agent (4). The comparative water absorbing agent (4) was evaluated in the same manner as in Example 1. Results are shown in Tables 2 to 4. Further, fluidity after moisture absorption was evaluated. Results are shown in Table 5. By applying the water spray mixing process and the curing process, the comparative water absorbing agent (4) obtained was in agglomelated state.

[Comparative Example 5]

The same procedures as in Example 4 were repeated except for using the water-absorbing resin (11) obtained in Reference Example 11 instead of the water-absorbing resin (4) obtained in Reference Example 4, to obtain a comparative water absorbing agent (5). The comparative water absorbing agent (5) was evaluated in the same manner as in Example 1. Results are shown in Tables 2 to 4. By applying the water spray mixing process and the curing process, the comparative water absorbing agent (5) obtained was in agglomerated state.

[Example 9]

To 100 parts by weight of the water absorbing agent (1) obtained in Example 1, 0.3 part by weight of fine particulate aluminum stearate (made by Kanto Chemical Co. , Inc.) was added and mixed (dry blend) to obtain a water absorbing agent (9). From measurement of particle size distribution of thus obtained a water absorbing agent (9), little change was found. Namely, mass median particle size (D50), logarithmic standard deviation ($\delta\zeta$) and percent by weight of particle diameter less than 150$\mu$m showed the same values as of the water absorbing agent (1) before mixing. Further, results of urine resistance test and extractables for 16 hours in a physiological saline solution also showed the same values as of the water absorbing agent (1) before mixing. Centrifuge retention capacity, absorbency against pressure at 1.9 kPa, extractables for 16 hours in a physiological saline solution, urine resistance, absorption speed, absorbency against pressure at 4. 8 kPa and fluidity after moisture absorption of the water absorbing agent (9) were measured. Results are shown in Table 5.

[Example 10]

To 100 parts by weight of the water absorbing agent (2) obtained in Example 2, 0.3 part by weight of fine particulate silicon dioxide (Trade Name: Aerosil 200 (average particle size of the primary particle: 12nm), made by Nippon Aerosil Co. , Ltd.) was added and mixed (dry blend) to obtain a water absorbing agent (10). From measurement of particle size distribution of thus obtained a water absorbing agent (10), little change was found. Namely, mass median particle size (D50), logarithmic standard deviation ($\delta\zeta$) and percent by weight of particle diameter less than 150$\mu$m showed the same values as of the water absorbing agent (2) before mixing. Further, results of urine resistance test and extractables for 16 hours in a physiological saline solution also showed the same values as of the water absorbing agent (2) before mixing. The water absorbing agent (10) was evaluated in the same manner as in Example 9. Results are shown in Table 5.

[Example 11]

The same procedures as in Example 10 were repeated except for using fine particulate aluminum stearate instead of the fine particulate silicon dioxide to obtain a water absorbing agent (11). From measurement of particle size distribution of thus obtained a water absorbing agent (11), little change was found. Namely, mass median particle size (D50), logarithmic standard deviation ($\delta\zeta$) and percent by weight of particle diameter less than 150$\mu$m showed the same values as of the water absorbing agent (2) before mixing. Further, results of urine resistance test and extractables for 16 hours in a physiological saline solution also showed the same values as of the water absorbing agent (2) before mixing. The water absorbing agent (11) was evaluated in the same manner as in Example 9. Results are shown in Table 5.

[Example 12]

The same procedures as in Example 10 were repeated except for using the water absorbing agent (3) obtained in Example 3 instead of the water absorbing agent (2) obtained in Example 2, to obtain a water absorbing agent (12). From measurement of particle size distribution of thus obtained a water absorbing agent (12), little change was found. Namely, mass median particle size (D50), logarithmic standard deviation ($\delta\zeta$) and percent by weight of particle diameter less than 150$\mu$m showed the same values as of the water absorbing agent (3) before mixing. Further, results of urine resistance test and extractables for 16 hours in a physiological saline solution also showed the same values as of the water absorbing agent (3) before mixing. The water absorbing agent (12) was evaluated in the same manner as in Example 9. Results are shown in Table 5.

[Example 13]

The same procedures as in Example 10 were repeated except for using the water absorbing agent (3) obtained in Example 3 instead of the water absorbing agent (2) obtained in Example 2 and fine particulate aluminum stearate instead of the fine particulate silicon dioxide, to obtain a water absorbing agent (13). From measurement of particle size distribution of thus obtained a water absorbing agent (13), little change was found. Namely, mass median particle size (D50), logarithmic standard deviation ($\delta\zeta$) and percent by weight of particle diameter less than 150$\mu$m showed the same values as of the water absorbing agent (3) beforemixing. Further, results of urine resistance test and extractables for 16 hours in a physiological saline solution also showed the same values as of the water absorbing agent (3) before mixing. The water absorbing agent (13) was evaluated in the same manner as in Example 9. Results are shown in Table 5.

[Example 14]

To 100 parts by weight of the water absorbing agent (4) obtained in Example 4, 0. 3 part by weight of fine particulate silicon dioxide (Trade Name: Aerosil 200) was added and mixed (dry blend) to obtain a water absorbing agent (14). From measurement of particle size distribution of thus obtained a water absorbing agent (14), littlechangewasfound. Namely, mass median particle size (D50), logarithmic standard deviation ($\delta\zeta$) and percent by weight of particle diameter less than 150$\mu$m showed the same values as of the water absorbing agent (4) before mixing. Further, results of urine resistance test and extractables for 16 hours in a physiological saline solution also showed the same values as of the water absorbing agent (4) before mixing. The water absorbing agent (14) was evaluated in the same manner as in Example 9. Results are shown in Table 5.

[Example 15]

To 100 parts by weight of the water absorbing agent (5) obtained in Example 5, 0.3 parts by weight of fine particulate magnesium stearate (made by Kanto Chemical Co., Inc.) was added and mixed (dry blend), to obtain a water absorbing agent (15). From measurement of particle size distribution of Thus obtained a water absorbing agent (15), little change was found. Namely, mass median particle size (D50), logarithmic standard deviation ($\delta\zeta$) and percent by weight of particle diameter less than 150$\mu$m showed the same values as of the water absorbing agent (5) before mixing. Further, results of urine resistance test and extractables for 16 hours in a physiological saline solution also showed the same values as of the water absorbing agent (5) before mixing. The water absorbing agent (15) was evaluated in the same manner as in Example 9. Results are shown in Table 5.

[Examples 16 to 18]

The same procedures as in Example 10 were repeated except for using the water absorbing agents (6) to (8) obtained in Examples 6 to 8 instead of the water absorbing agent (2) obtained in Example 2 to obtain a water absorbing agents (16) to (18), respectively. From measurements of particle size distributions of thus obtained a water absorbing agents (16) to (18), little change was found in any agent. Namely, mass median particle size (D50), logarithmic standard deviation ($\delta\zeta$) and percent by weight of particle diameter less than 150$\mu$m of each agent showed the same values as of the corresponding a water absorbing agents (6) to (8) before mixing, respectively. Further, results of urine resistance test and extractables for 16 hours in a physiological saline solution of each agent also showed the same values as of the corresponding a water absorbing agent (6) to (8) before mixing, respectively. The water absorbing agents (16) to (18) were evaluated in the same manner as in Example 9. Results are shown in Table 5.

[Comparative Examples 6 and 7]

The same procedures as in Example 10 were repeated except for using the comparative water absorbing agents (1) and (2) obtained in Comparative Examples 1 and 2 instead of the water absorbing agent (2) obtained in Example 2, to obtain a comparative water absorbing agents (6) and (7), respectively. From measurements of particle size distributions of thus obtained comparative water absorbing agents (6) and (7), little change was found in any agent. Namely, mass median particle size (D50), logarithmic standard deviation ($\delta\zeta$) and percent by weight of particle diameter less than 150$\mu$m of each agent showed the same values as of the corresponding a water absorbing agents (1) and (2) before mixing, respectively. Further, results of urine resistance test and extractables for 16 hours in a physiological saline solution of each agent also showed the same values as of the, corresponding a comparative water absorbing agent (1) and (2) before mixing, respectively. Further, results of urine resistance test and extractables for 16 hours in a physiological saline solution of each agent also showed the same values as of the corresponding a comparative water absorbing agent (1) and (2) before mixing, respectively. The comparative water absorbing agents (6) and (7) were evaluated in the same manner as in Example 9. Results are shown in Table 5.

Further, fluidity after moisture absorption of the comparative water absorbing agents (3) and (4) were also measured. Results are shown in Table 5.

[Example 19]

To 100 parts by weight of the water-absorbing resin (2) obtained in Reference Example 2, 2 parts by weight of an aqueous solution comprising sodium diethylenetriamine pentaacetate aqueous solution and a 15% by weight aqueous solution of extract from plant leaves of Theaceae plant (Trade Name: FS-80MO, Supplier: Shiraimatu Shinyaku Co., Ltd., Address : 37-1 Ugawa, Mizuguchi-Cho, kouga-Gun, Shiga-Ken, Japan)(amount of each component was adjusted so that sodium diethylenetriamine pentaacetate and the 15% by weight aqueous solution of extract from plant leaves of Theaceae plant became 50 ppm and 0.1% by weight relative to the water-absorbing resin (2), respectively) was mixed by spraying. The resultant mixture was cured at 60°C for 1 hour to obtain a water absorbing agent (19). Particle size distribution of thus obtained a water absorbing agent (19) was the same as in Example 10 wherein the 15% by weight aqueous solution of extract from plant leaves of Theaceae plant was not added. Further, centrifuge retention capacity, absorbency against pressure at 1.9 kPa, deodorization test, extractables for 16 hours in a physiological saline solution, urine resistance and absorbency against pressure at 4. 8 kPa of the water absorbing agent (19) were measured. Results are shown in Table 6.

[Example 20]

The same procedures as in Example 10 were repeated except for using zinc and silicon composite hydrated oxide (Trade Name: CERATIOX SZ-100, made by Titan Kogyo K. K. , weight ratio of Zn and Si contents: 82/18, average particle size: 0.36μm) instead of fine particulate silicon dioxide, to obtain a water absorbing agent (20). Particle size distribution of thus obtained a water absorbing agent (20) was the same as of the water absorbing agent (10). Further, the water absorbing agent (20) was evaluated in the same manner as the water absorbing agent (19). Results are shown in Table 6.

[Example 21]

The same procedures as in Example 10 were repeated except for using the water-absorbing resin (3) obtained in Reference Example 3 instead of the water-absorbing resin (2) obtained in Reference Example 2, to obtain a water absorbing agent (21). Particle size distribution of thus obtained a water absorbing agent (21) was the same as of the water absorbing agent (12). Further, the water absorbing agent (21) was evaluated in the same manner as the water absorbing agent (19). Results are shown in Table 6.

[Example 22]

The same procedures as in Example 12 were repeated except for using zinc/silicon composite hydrated oxide (Trade Name: CERATIOX SZ-100, made by Titan Kogyo K.K., weight ratio of Zn and Si contents: 82/18, average particle size: 0.36μm) instead of fine particulate silicon dioxide to obtain a water absorbing agent (22). Particle size distribution of thus obtained a water absorbing agent (22) was the same as of the water absorbing agent (12). Further, the water absorbing agent (22) was evaluated in the same manner as the water absorbing agent (19). Results are shown in Table 6.
Further, results of deodorization tests of the comparative water absorbing agents (3) and (4) are shown together in Table 6.

[Example 23]

To evaluate an absorbing substrate performance of the water absorbing agent (2) obtained in Example 2, an absorbing substrate for evaluation (1) was prepared according to the method (j) for evaluation of an absorbing substrate performance described above, to measure rewet amount after 10 min. and rewet amount after deterioration. Results are shown in Table 7.

[Examples 24 to 27]

The same procedures as in Example 23 were repeated except for using the water absorbing agents (13) and (16) to (18) obtained in Examples 13 and 16 to 18 instead of the water absorbing agent (2) used in Example 23, to obtain absorbing substrates for evaluation (2) to (5), respectively.
Evaluation results on rewet amounts of thus obtained absorbing articles (2) to (5) are shown in Table 7.

[Comparative Examples 8 to 12]

The same procedures as in Example 23 were repeated except for using the comparative water absorbing agents (3) to (7) obtained in Comparative Examples 3 to 7 instead of the water absorbing agent (2) used in Example 23 to obtain absorbing substrates for comparative evaluation (1) to (5), respectively.

Evaluation results on rewet amounts of thus obtained absorbing substrates for comparative evaluation (1) to (5) are shown in Table 7.

[Example 28]

To 1,500g of an acrylic acid aqueous solution (monomer concentration 20%), 3.1g of N,N'-methylenebisacrylamide was dissolved to prepare a reaction solution, and the resultant reaction solution was charged into a stainless steel tray used in Reference Example 1, so that thickness of the reaction solution became 17 mm. The stainless steel tray, after sealed in the same manner as in Reference Example 1, was dipped into a water bath at 20 °C, and nitrogen gas was introduced through the reaction solution to remove dissolved oxygen in the solution, while adjusting temperature of the reaction solution at 20°C. After that, nitrogen gas was introduced intro the upper space of the reactor, and kept exhausting from the opposite side. Then, 20.0g of a 10% by weight 2,2'-azobis(2-amidinopropane)dihydrochloride aqueous solution, 18.0g of a 1% by weight L-ascorbic acid aqueous solution and 20.0g of a 3.5% by weight hydrogen peroxide aqueous solution as polymerization initiators were added, while stirring the reaction solution with magnetic stirrer, to start polymerization about 1 minute later. By repeating cooling and heating from the bottom surface of the bath during the polymerization reaction, the solution showed peak temperature of 60°C after 12 minutes from the start of the polymerization. A hydrated gel-like polymer was taken out after 100 minutes from the start of the polymerization. Thus obtained hydrated gel-like polymer was cut off into about 5cm square pieces with scissors, which were fed into the same meat chopper as used in Reference Example 1 at constant rate, together with 749g of a 40% by weight sodium hydroxide aqueous solution, also being fed at constant rate, to crush and post-neutralize the gel simultaneously. The crushed gel discharged from the meat chopper was maintained in atmosphere at about 70°C until the gel did not indicate red color even by the addition of a phenolphthalein indicator, followed by drying and pulverizing in the same manner as in Reference Example 1, further classification by metal sieves with mesh opening size with of 710 $\mu$m and 150$\mu$m and to obtain a water-absorbing resin powder (1) with irregularly pulverized shape. Centrifuge retention capacity (CRC), mass median particle size (D50) and percent by weight of particles less than 150$\mu$m (%) of the water-absorbing resin (1) obtained are shown in Table 1.

Next, to 100 parts by weight of the water-absorbing resin powder (1) obtained, 3.53 parts by weight of a surface crosslinking agent consisting of 0.5 part by weight of propyleneglycol, 0.03 part by weight of ethyleneglycol glycidylether, 0.3 part by weight of 1,4-butandiol and 2.7 parts by weight of water were mixed. The mixture was heat treated at 195°C of heating medium temperature for 60 minutes to obtain water-absorbing resin (12).

Thus obtained water-absorbing resin (12) was used as it is as a water absorbing agent (23), which was evaluated in the same manner as in Example 1. Results are shown in Tables 2 to 4.

[Example 29]

To 100 parts by weight of the water absorbing agent (23) obtained in Example 28, 0.1 part by weight of fine particulate calcium stearate (made by NOF Corp.) was added and mixed (dry blend) to obtain a water absorbing agent (24). From measurement of particle size distribution of thus obtained a water absorbing agent (24), little change was found. Namely, mass median (D50), logarithmic standard deviation ($\delta\zeta$) and percent by weight of particle diameter less than 150$\mu$m showed the same values as of the water absorbing agent (23) before mixing. Further, results of urine resistance test and extractables for 16 hours in a physiological saline solution also showed the same values as of the water absorbing agent (23) before mixing. The water absorbing agent (23) was evaluated in the same manner as in Example 9. Results are shown in Table 5.

[Example 30]

To evaluate absorbing substrate performance of the water absorbing agent (23) obtained in Example 28, an absorbing substrate for evaluation (6) was prepared according to the method (j) for evaluation of the absorbing substrate performance, to measure rewet amount after 10 minutes and rewet amount after deterioration. Results are shown in Table 7.

Table 1

| | Water-Absorbing Resin Powder | CRC (g/g) | Mass Median Particle size ($\mu$m) | Ratio of particle smaller than 150$\mu$m (%) |
|---|---|---|---|---|
| Reference Example 1 | Water-Absorbing resin powder (a) | 42 | 370 | 1.5 |
| Reference Example 2 | Water-Absorbing resin powder (b) | 45 | 360 | 1.5 |
| Reference Example 3 | Water-Absorbing resin powder (c) | 57 | 360 | 1.6 |
| Reference Example 4 | Water-Absorbing resin powder (d) | 48 | 310 | 1.5 |
| Reference Example 5 | Water-Absorbing resin powder (e) | 52 | 355 | 1.7 |
| Reference Example 6 | Water-Absorbing resin powder (f) | 50 | 310 | 1.4 |
| Reference Example 7 | Water-Absorbing resin powder (g) | 46 | 225 | 1.5 |
| Reference Example 8 | Water-Absorbing resin powder (h) | 57 | 230 | 1.6 |
| Reference Example 9 | Water-Absorbing resin powder (i) | 45 | 350 | 5.0 |
| Reference Example 10 | Water-Absorbing resin powder (j) | 56 | 495 | 0.5 |
| Reference Example 11 | Water-Absorbing resin powder (k) | 32 | 355 | 1.5 |
| Example 28 | Water-Absorbing resin powder (1) | 48 | 370 | 0.5 |
| EX. :Example, Comp.EX. :Comparative Example, WA :Water absorbing agent, Comp. WA :Comparative Water absorbing agent, CRC : Centrifuge Retention Capacity, AAP :Absorbency Against Pressure | | | | |

Table 2

| | Water absorbing agent | Not smaller than 850μm (wt%) | Not smaller than 710μm Smaller than 850μm (Wt%) | Not smaller than 600μm Smaller than 710μm (Wt%) | Not smaller than 500μm Smaller than 600μm (W%) | Not smaller than 425μm Smaller than 500μm (met%) | Not smaller than 300μm Smaller than 425μm (Wt%) | Not smaller than 212μm Smaller than 300μm (Wt%) | Not smaller than 150μm Smaller than 212μm (Wt%) | Not smaller than 45μm Smaller than 150μm (Wt%) | Smaller than 45μm (Wt%) |
|---|---|---|---|---|---|---|---|---|---|---|---|
| EX. 1 | WA (1) | 0.0 | 0.0 | 5.0 | 15.3 | 18.3 | 30.1 | 20.0 | 9.8 | 1.3 | 0.2 |
| EX. 2 | WA (2) | 0.0 | 0.0 | 2.3 | 16.3 | 18.6 | 33.3 | 19.9 | 8.9 | 0.6 | 0.1 |
| EX. 3 | WA (3) | 0.0 | 0.0 | 2.3 | 15.3 | 19.4 | 33.5 | 19.8 | 8.9 | 0.8 | 0.0 |
| EX. 4 | WA (4) | 0.0 | 0.0 | 0.0 | 1.1 | 15.6 | 42.6 | 28.1 | 11.3 | 1.1 | 0.2 |
| EX. 5 | WA (5) | 0.0 | 0.0 | 0.1 | 10.6 | 19.4 | 46.5 | 18.2 | 3.7 | 1.4 | 0.1 |
| EX. 6 | WA (6) | 0.0 | 0.0 | 0.0 | 5.4 | 17.4 | 40.9 | 29.4 | 5.5 | 1.1 | 0.3 |
| EX. 7 | WA (7) | 0.0 | 0.0 | 0.0 | 0.0 | 0.6 | 11.4 | 59.8 | 26.8 | 1.2 | 0.2 |
| EX. 8 | WA (8) | 0.0 | 0.0 | 0.0 | 0.0 | 6.6 | 11.7 | 55.8 | 24.5 | 1.2 | 0.2 |
| Comp. EX. 1 | Comp.WA (1) | 0.0 | 1.1 | 5.5 | 15.6 | 13.9 | 33.7 | 17.1 | 9.7 | 3.3 | 0.1 |
| Comp. EX. 2 | Comp.WA (2) | 0.1 | 4.0 | 27.2 | 20.6 | 10.6 | 23.0 | 11.3 | 2.5 | 0.6 | 0.1 |
| Comp. EX. 3 | Comp.WA (3) | 0.0 | 0.0 | 2.8 | 16.4 | 18.7 | 33.5 | 18.7 | 9.1 | 0.7 | 0.1 |
| Comp. EX. 4 | Comp.WA (4) | 0.0 | 0.0 | 4.2 | 14.9 | 19.7 | 34.5 | 17.6 | 8.5 | 0.5 | 0.1 |
| Comp. EX. 5 | Comp.WA (5) | 0.0 | 0.0 | 0.0 | 2.3 | 15.8 | 43.1 | 25.2 | 12.3 | 1.0 | 0.3 |
| EX. 28 | WA (23) | 0.0 | 0.0 | 1.9 | 13.9 | 20.5 | 36.8 | 18.5 | 7.6 | 0.8 | 0.0 |
| EX. :Example, Comp.EX. :Comparative Example, WA :Water absorbing agent, Comp. WA :Comparative Water absorbing agent | | | | | | | | | | | |

Table 3

| | Water absorbing agent | mass median particle size D50 ($\mu$m) | Logarithmic Standard Deviation ($\delta\zeta$) |
|---|---|---|---|
| Example 1 | Water absorbing agent (1) | 373 | 0.397 |
| Example 2 | Water absorbing agent (2) | 373 | 0.369 |
| Example 3 | Water absorbing agent (3) | 372 | 0.367 |
| Example 4 | Water absorbing agent (4) | 321 | 0.322 |
| Example 5 | Water absorbing agent (5) | 367 | 0.281 |
| Example 6 | Water absorbing agent (6) | 335 | 0.298 |
| Example 7 | Water absorbing agent (7) | 238 | 0.201 |
| Example 8 | Water absorbing agent (8) | 245 | 0.241 |
| Comparative Example 1 | Comparative Water Absorbing Agent (1) | 369 | 0.423 |
| Comparative Example 2 | Comparative Water absorbing agent (2) | 508 | 0.367 |
| Comparative Example 3 | Comparative Water absorbing agent (3) | 376 | 0.371 |
| Comparative Example 4 | Comparative Water absorbing agent (4) | 381 | 0.361 |
| Comparative Example 5 | Comparative Water absorbing agent (5) | 326 | 0.333 |
| Example 28 | Water absorbing agent (23) | 375 | 0.343 |

Table 4

| | Water absorbing agent | CRC (g/g) | AAP at 1.9kPa (g/g) | Extractables for 16 Hours in Saline Soln. (%) | Increased Extractables by Deterioration (%) | Increased Ratio of Extractables by Deterioration | Absorption speed (sec.) | AAP at 4.8kPa (g/g) | Extractables for 1 hour in Deterioration Test Liquid (%) |
|---|---|---|---|---|---|---|---|---|---|
| EX. 1 | WA (1) | 33 | 33 | 8 | 14 | 3.8 | 45 | 25 | 19 |
| EX. 2 | WA (2) | 35 | 33 | 18 | 2 | 1.2 | 46 | 23 | 11 |
| EX. 3 | WA (3) | 43 | 38 | 30 | 2 | 1.2 | 42 | 15 | 13 |
| EX. 4 | WA (4) | 37 | 32 | 23 | 14 | 2.1 | 42 | 22 | 27 |
| EX. 5 | WA (5) | 41 | 30 | 32 | 13 | 2.0 | 44 | 16 | 26 |
| EX. 6 | WA (6) | 45 | 35 | 8 | 9 | 4.0 | 43 | 19 | 12 |
| EX. 7 | WA (7) | 35 | 33 | 18 | 5 | 1.5 | 32 | 23 | 15 |
| EX. 8 | WA (8) | 45 | 37 | 30 | 3 | 1.3 | 29 | 15 | 14 |
| Comp. EX. 1 | Comp. WA (1) | 35 | 33 | 18 | 3 | 1.4 | 48 | 24 | 11 |
| Comp. EX. 2 | Comp. WA (2) | 42 | 33 | 30 | 2 | 1.2 | 63 | 14 | 12 |
| Comp. EX. 3 | Comp. WA (3) | 35 | 33 | 18 | 41 | 5.6 | 46 | 23 | 50 |
| Comp. EX. 4 | Comp. WA (4) | 43 | 34 | 30 | 59 | 6.4 | 45 | 15 | 70 |
| Comp. EX. 5 | Comp. WA (5) | 29 | 23 | 5 | 13 | 5.3 | 50 | 27 | 16 |
| EX. 28 | WA (23) | 38 | 35 | 2 | 8 | 5.0 | 43 | 26 | 10 |
| EX. :Example, Comp.EX. :Comparative Example, WA :Water absorbing agent, Comp. WA :Comparative Water absorbing agent CRC : Centrifuge Retention Capacity, AAP :Absorbency Against Pressure | | | | | | | | | |

Table 5

| | Water absorbing, agent | CRC (g/g) | AAP at 1.9kPa (g/g) | Extractab les for 16 Hours in Saline Soln. (%) | Increased Extractab les by Deterioration (%) | Increased Ratio of Extractab les by Deterioration | Absorption Speed (sec.) | Fluidity after moisture absorption (%) | AAP at 4.8kPa (g/g) | Extractables for 1 hour in Deterioration Test Liquid (%) |
|---|---|---|---|---|---|---|---|---|---|---|
| EX. 9 | WA(9) | 33 | 33 | 8 | 14 | 3.8 | 48 | 0 | 25 | 19 |
| EX. 10 | WA(10) | 35 | 30 | 18 | 2 | 1.2 | 40 | 0 | 19 | 11 |
| EX. 11 | WA(11) | 35 | 33 | 18 | 2 | 1.2 | 49 | 0 | 23 | 11 |
| EX. 12 | WA(12) | 43 | 29 | 30 | 2 | 1.2 | 34 | 0 | 9 | 13 |
| EX. 13 | WA(13) | 43 | 38 | 30 | 2 | 1.2 | 45 | 0 | 15 | 13 |
| EX. 14 | WA14) | 37 | 29 | 23 | 14 | 2.1 | 38 | 0 | 15 | 27 |
| EX. 15 | WA(15) | 41 | 30 | 32 | 13 | 2.0 | 47 | 0 | 16 | 26 |
| EX. 16 | WA(16) | 45 | 24 | 8 | 9 | 4.0 | 35 | 0 | 9 | 3 |
| EX. 17 | WA(17) | 34 | 30 | 18 | 5 | 1.5 | 25 | 0 | 18 | 15 |
| EX. 18 | WA(18) | 44 | 28 | 30 | 3 | 1.3 | 25 | 0 | 8 | 14 |
| Comp. EX. 3 | Comp. WA (3) | 35 | 33 | 18 | 41 | 5.6 | 46 | 70 | 23 | 50 |
| Comp. EX. 4 | Comp. WA (4) | 43 | 34 | 30 | 59 | 6.4 | 45 | 50 | 15 | 70 |
| Comp. EX. 6 | Comp. WA (6) | 35 | 30 | 18 | 3 | 1.4 | 44 | 0 | 17 | 11 |
| Comp. EX. 7 | Comp. WA (7) | 42 | 25 | 30 | 2 | 1.2 | 60 | 0 | 8 | 12 |
| EX. 29 | WA(24) | 38 | 35 | 26 | 8 | 5.0 | 47 | 0 | 26 | 10 |

EX. :Example, Comp.EX. :Comparative Example, WA :Water absorbing agent, Comp. WA :Comparative Water absorbing agent CRC : Centrifuge Retention Capacity, AAP :Absorbency Against Pressure

Table 6

| | Water absorbing agent | Centrifuge Retention Capacity (g/g) | Absorbency Against Pressure at 1.9kPa (g/g) | Result of Deodorization Test | Extractables for 16 Hours in Saline Soln. (%) | Increased Extractables by Deterioration (%) | Increased Ratio of Extractables by Deterioration | Absorbency Against Pressure at 4.8kPa (g/g) | Extractables for 1 Hour in Saline Soln. (%) |
|---|---|---|---|---|---|---|---|---|---|
| Ex. 19 | WA (19) | 33 | 33 | 2.5 | 18 | 2 | 1.2 | 23 | 11 |
| Ex. 20 | WA (20) | 35 | 30 | 2.6 | 18 | 2 | 1.2 | 19 | 11 |
| Ex. 21 | WA (21) | 43 | 29 | 2.6 | 30 | 2 | 1.2 | 15 | 13 |
| Ex. 22 | WA (22) | 43 | 29 | 2.7 | 30 | 2 | 1.2 | 9 | 13 |
| Comp. Ex. 3 | Comp.WA (3) | 35 | 33 | 4.8 | 18 | 41 | 5.6 | 23 | 50 |
| Comp. Ex. 4 | Comp.WA (4) | 43 | 34 | 4.9 | 30 | 59 | 6.4 | 15 | 70 |

EX. :Example, Comp.EX. :Comparative Example, WA :Water absorbing agent, Comp. WA :Comparative Water absorbing agent

Table 7

| | Absorbing Substrate | Water absorbing agent Used | Rewet Amount after 10 Min. | Rewet Amount after Deterioration |
|---|---|---|---|---|
| Example 23 | Absorbing Substrate for Evaluation (1) | Water absorbing agent (2) | 8 | 6 |
| Example 24 | Absorbing Substrate for Evaluation (2) | Water absorbing agent (13) | 6 | 1 |
| Example 25 | Absorbing Substrate for Evaluation (3) | Water absorbing agent (16) | 4 | 3 |
| Example 26 | Absorbing Substrate for Evaluation (4) | Water absorbing agent (17) | 8 | 6 |
| Example 27 | Absorbing Substrate for Evaluation (5) | Water absorbing agent (18) | 3 | 1 |
| Comparative Example 8 | Absorbing Substrate for Comparative Evaluation (1) | Comparative Water absorbing agent (3) | 8 | 17 |
| Comparative Example 9 | Absorbing Substrate for Comparative Evaluation (1) | Comparative Water absorbing agent (4) | 6 | 20 |
| Comparative Example 10 | Absorbing Substrate for Comparative Evaluation (1) | Comparative Water absorbing agent (5) | 12 | 10 |
| Comparative Example 11 | Absorbing Substrate for Comparative Evaluation (1) | Comparative Water absorbing agent (6) | 10* | 6 |
| Comparative Example 12 | Absorbing Substrate for Comparative Evaluation (1) | Comparative Water absorbing agent (7) | 10 | 1 |
| Example 30 | Absorbing Substrate for Evaluation (1) | Water absorbing agent (23) | 7 | 2 |
| *: After measurement of rewet amount after 10 Min., fine gel particles were observed on nonwoven fabric as gel on skin. | | | | |

A particulate water absorbing agent of the present invention has, as shown in Tables 2 to 4, a well controlled particle size, high absorption capacity, little difference between extractabless in physiological saline solutions containing and not containing L-ascorbic acid, and thereby shows stable high performance, irrespective of changes in urine compositions (individual difference, seasonal variation, and the like) and time in use.

A particulate water absorbing agent of the present invention is, as shown in Table 5, superior in absorption speed and fluidity after moisture absorption, and shows high deodorization performance by adding a deodorant, if necessary, as separately shown in Table 6.

A particulate water absorbing agent of the present invention provides, as shown in Table 7, absorbing articles (absorbing substrates in Table 7) having small rewet amount for any kind of liquid. Further, a particulate water absorbing agent of the present invention does not bring about leak-out of fine gel particles from an absorbing substrate, and thereby provides absorbing articles (diapers) having stable and high performance, irrespective of changes in urine compositions (individual difference, seasonal variation, and the like) and time in use.

Industrial Applicability

A water-absorbing resin obtained according to the present invention has well controlled specific particle size distribution and superior stability against urine, and exerts effect that it can provide an absorbing substrate having very superior absorbing performance in comparison with a conventional absorbing substrate, when used for an absorbing substrate such as a diaper.

**Claims**

1. A particulate water absorbing agent comprising a water-absorbing resin obtained by crosslinking polymerization of an unsaturated monomer containing an acid group and/or a salt thereof, wherein said water-absorbing resin is further surface crosslinked, and wherein said water absorbing agent satisfies the following (a) to (d) and (1):

    (a) centrifuge retention capacity (CRC) of the water absorbing agent in a physiological saline solution being not lower than 32 g/g;
    (b) mass median particle size (D50) of the water absorbing agent being in the range of 200 to 400 $\mu$m;
    (c) ratio of particles of the water absorbing agent having diameter of smaller than 150 $\mu$m being in the range of 0 to 2% by weight;
    (d) increased extractables of the water absorbing agent by deterioration expressed by the following formula (I) of 0 to 15% by weight and extractables of the water absorbing agent for one hour in deterioration test liquid of 0.1 to 30% by weight, wherein the deterioration test liquid means a physiological saline solution containing 0.05% by weight of L-ascorbic acid;

    formula (I):

    Increased extractables by deterioration (% by weight)

    = extractables for one hour in deterioration test liquid (% by weight)

    - extractables for one hour in a physiological saline solution (% by

    weight);

    and
    (l) logarithmic standard deviation of particle size distribution being in the range of 0.20 to 0.40.

2. A particulate water absorbing agent, according to claim 1, wherein the water absorbing agent satisfies further (e):

    (e) increased ratio of extractables of the water absorbing agent by deterioration expressed by the following formula (II) of 1 to 4 times, and extractables of the water absorbing agent for one hour in deterioration test liquid of 0.1 to 30% by weight, wherein the deterioration test liquid means a physiological saline solution containing 0.05% by weight of L-ascorbic acid;

    formula (II):

    Increased ratio of extractables by deterioration = extractables for one hour in deterioration test liquid (% by

    weight)/extractables for one hour in a physiological saline solution

    (% by weight).

3. A particulate water absorbing agent, according to claims 1 or 2, wherein the water absorbing agent satisfies further (f) and (g):

    (f) extractables of the water absorbing agent for 16 hours in a physiological saline solution being in the range of 0.1 to 10% by weight;
    (g) absorbency against pressure at 4.8 kPa (AAP4.8 kPa) of the absorbent in a physiological saline solution being not lower than 21 g/g, preferably being not lower than 22 g/g.

4. A particulate water absorbing agent according to any one of claims 1 to 3, wherein a water-absorbing resin is surface crosslinked using polyvalent alcohol as a surface crosslinking agent.

**5.** A particulate water absorbing agent according to any one of claims 1 to 4, wherein further (h) 90 to 100% by weight of a particulate water absorbing agent has diameter in the range of 600 to 150 $\mu$m.

**6.** A particulate water absorbing agent according to any one of claims 1 to 5, wherein the water absorbing agent satisfies further (i) absorbency against pressure at 1.9 kPa (AAP1.9 kPa) in a physiological saline solution being not lower than 25 g/g.

**7.** A particulate water absorbing agent according to any one of claims 1 to 6, wherein the water absorbing agent satisfies further (j) absorption speed with vortex method being not longer than 60 seconds.

**8.** A particulate water absorbing agent according to any one of claims 1 to 7, wherein the water absorbing agent satisfies further (k) fluidity after moisture absorption being in the range of 0 to 20% by weight.

**9.** A particulate water absorbing agent according to any one of claims 1 to 8, which further comprises, besides a water-absorbing resin as a main component, one or more minor components selected from the group consisting of a chelating agent, a deodorant, a polyvalent metal salt and an inorganic fine particle.

**10.** A particulate water absorbing agent according to claim 9, wherein the chelating agent is one or more agents selected from the group consisting of aminocarboxylic acid and salt thereof.

**11.** A particulate water absorbing agent according to claim 9, wherein the deodorant is a component made from a plant.

**12.** A particulate water absorbing agent according to claim 9, wherein the polyvalent metal salt is a polyvalent metal salt of an organic acid.

**13.** A particulate water absorbing agent according to claim 9, wherein the inorganic fine particle is a composite hydrated oxide.

**14.** A particulate water absorbing agent according to any one of claims 1 to 13, wherein particle shape of a water-absorbing resin is irregularly pulverized shape.

**15.** A particulate water absorbing agent according to any one of claims 1 to 14, wherein the above (d) increased extractables by deterioration is 0 to 12% by weight.

**16.** A particulate water absorbing agent according to any one of claims 2 to 15, wherein the above (e) increased ratio of extractables by deterioration is 1 to 3.

**17.** An absorbing article for excrement, urine or blood, which is molded using a particulate water absorbing agent according to any one of claims 1 to 16 and hydrophilic fibers.

**18.** An absorbing article for excrement, urine or blood according to claim 17, wherein, a content of the water absorbing agent in the absorbing article, based on total weight of the water absorbing agent and hydrophilic fibers is 20 to 100%.

**19.** A method for production of a particulate water absorbing agent according to any one of claims 1 to 16, which comprises

a step of crosslinking polymerization of the aqueous solution of an unsaturated monomer containing non-neutralized acrylic acid and/or salts thereof in the presence of a crosslinking agent and a chain transfer agent in an amount of 0.001% to 1% by mole based on total monomers;
a step of adjustment to particle size distribution; and
a step of surface crosslinking the water-absorbing resin particle obtained by the polymerization and satisfying (a) to (c) described below,

(a) centrifuge retention capacity (CRC) of the resin particle in a physiological saline solution being in the range of not lower than 32 g/g;
(b) mass median particle size (D50) of the resin particle being in the range of 200 to 400 $\mu$m; and
(c) ratio of particles having diameter of the resin particle of smaller than 150 $\mu$m being in the range of 0 to 2% by weight.

**20.** A method for production of a particulate water absorbing agent according to any one of claims 1 to 16, which comprises

a step of crosslinking polymerization of the aqueous solution of an unsaturated monomer in concentration of 10 to 30% by weight containing non-neutralized acrylic acid in the presence of a crosslinking agent; a step of neutralization after the polymerization; and a step of surface crosslinking the water-absorbing resin particle obtained by the neutralization and satisfying (a) to (c) described below,

(a) centrifuge retention capacity (CRC) of the resin particle in a physiological saline solution being in the range of not lower than 32 g/g; (b) mass median particle size (D50) of the resin particle being in the range of 200 to 400 $\mu$m; and (c) ratio of particles having diameter of the resin particle of smaller than 150 $\mu$m being in the range of 0 to 2% by weight.

**21.** A method for production of a particulate water absorbing agent according to any one of claims 1 to 16, which comprises

a step of crosslinking polymerization of the aqueous solution of an unsaturated monomer containing non-neutralized acrylic acid and/or salts thereof in the presence of a crosslinking agent; a step of surface crosslinking the water-absorbing resin particle obtained by the polymerization and satisfying (a) to (c) described below,

(a) centrifuge retention capacity (CRC) of the resin particle in a physiological saline solution being in the range of not lower than 32 g/g; (b) mass median particle size (D50) of the resin particle being in the range of 200 to 400 $\mu$m; (c) ratio of particles having diameter of the resin particle of smaller than 150 $\mu$m being in the range of 0 to 2% by weight; and

a step of adding a chelating agent with one or more timings selected from the group consisting of

(i) during polymerization (ii) after the polymerization and before surface crosslinking (iii) during surface crosslinking (iv) after surface crosslinking.

**22.** A method for production according to any one of claims 19 to 21, wherein, the step of surface crosslinking is conducted by using polyvalent alcohol as a surface crosslinking agent.

**23.** A method for the production of a particulate water absorbing agent according to any one of claims 19-22, which further comprises a step of removing coarse particles having a diameter of 400 $\mu$m to 5000 $\mu$m and fine particles having a diameter of smaller than 150 $\mu$m from the water-absorbing resin particle prior to the surface crosslinking step.

**Patentansprüche**

**1.** Partikelförmiges wasserabsorbierendes Mittel, aufweisend ein wasserabsorbierendes Harz, erhältlich durch Vernetzungspolymerisation eines ungesättigten Monomers, enthaltend eine Säuregruppe und/oder ein Salz davon, wobei das wasserabsorbierende Harz weiterhin oberflächenvernetzt ist, und wobei das wasserabsorbierende Harz den Folgenden (a) bis (d) und (1) genügt:

(a) Zentrifugenretentionskapazität (CRC) des wasserabsorbierenden Mittels in einer physiologischen Salzlösung von nicht geringer als 32 g/g; (b) Massenmedian der Partikelgröße (D50) des wasserabsorbierenden Harzes im Bereich von 200 bis 400 $\mu$m; (c) Anteil von Partikeln des wasserabsorbierenden Mittels mit einem Durchmesser von kleiner als 150 $\mu$m im Bereich von 0 bis 2 Gew.-%; (d) Zunahme an extrahierbaren Substanzen des wasserabsorbierenden Mittels durch Alterung, ausgedrückt durch die folgende Formel (I) von 0 bis 15 Gew.-% und extrahierbare Substanzen des wasserabsorbierenden Mittels nach einer Stunde in Alterungstestflüssigkeit von 0,1 bis 30 Gew.-%, wobei die Alterungstestflüssigkeit eine physiologische Salzlösung meint, die 0,05 Gew.-% L-Ascorbinsäure enthält;

Formel (I):

Zunahme an Extrahierbaren durch Alterung (Gew.-%)

= Extrahierbare nach einer Stunde in Alterungstestflüssigkeit (Gew.-%)

- Extrahierbare nach einer Stunde in physiologischer Kochsalzlösung

(Gew.-%);

und

(l) die logarithmische Standardabweichung der Partikelgrößenverteilung ist im Bereich von 0,20 bis 0,40.

2. Partikelförmiges wasserabsorbierenden Mittel nach Anspruch 1, wobei das wasserabsorbierende Mittel weiterhin der Bedingung (e) genügt:

(e) Zunahme des Verhältnisses von Extrahierbaren des Wasser absorbierenden Mittels durch Alterung, ausgedrückt durch die folgende Formel (II) von 1 bis 4 mal, und Extrahierbare des Wasser absorbierendes Mittel nach einer Stunde in Alterungstestflüssigkeit von 0,2 bis 30 Gew.-%, wobei die Alterungstestflüssigkeit eine physiologische Salzlösung meint, die 0,05 Gew.-% L-Ascorbinsäure enthält;

Formel (II):

Zunahme an Extrahierbaren durch Alterung = Extrahierbare nach einer Stunde in Alterungstestflüssigkeit (Gew.-%) /

Extrahierbare nach einer Stunde in physiologischer Salzlösung (Gew.-%).

3. Partikelförmiges wasserabsorbierendes Mittel nach Anspruch 1 oder 2, wobei das wasserabsorbierende Mittel weiterhin (f) und (g) genügt:

(f) Extrahierbare des Wasser absorbierenden Mittels nach 16 Stunden in einer physiologischen Salzlösung im Bereich von 0,1 bis 10 Gew.-%;
(g) Absorption unter Druck bei 4,8 kPa (AAP4.8kPa) des Absorbens in einer physiologischen Salzlösung ist nicht kleiner als 21 g/g, vorzugsweise nicht kleiner als 22 g/g.

4. Partikelförmiges wasserabsorbierendes Mittel nach einem der Ansprüche 1 bis 3, wobei ein wasserabsorbierendes Harz unter Verwendung eines mehrwertigen Alkohols als Oberflächenvernetzungsmittel oberflächenvernetzt wird.

5. Partikelförmiges wasserabsorbierendes Mittel nach einem der Ansprüche 1 bis 4, wobei weiterhin (h) 90 bis 100 Gew.-% des partikelförmigen wasserabsorbierenden Mittels einen Durchmesser im Bereich von 600 bis 150 $\mu$m hat.

6. Partikelförmiges wasserabsorbierendes Harz nach einem der Ansprüche 1 bis 5, wobei das wasserabsorbierende Mittel weiterhin (i) genügt, einer Absorption unter Druck bei 1,9 kPa (AAP1.9kPa) in physiologischer Salzlösung von nicht kleiner als 25 g/g.

7. Partikelförmiges wasserabsorbierendes Mittel nach einem der Ansprüche 1 bis 6, wobei das wasserabsorbierende Mittel weiterhin (j) genügt, einer Absorptionsgeschwindigkeit nach dem Vortexverfahren von nicht länger als 60 Sekunden.

8. Partikelförmiges wasserabsorbierendes Harz nach einem der Ansprüche 1 bis 7, wobei das wasserabsorbierende Mittel weiterhin (k) genügt, einer Fluidität nach Feuchtigkeitabsorption im Bereich von 0 bis 20 Gew.-%.

9. Partikelförmiges wasserabsorbierendes Mittel nach einem der Ansprüche 1 bis 8, welches weiterhin neben einem wasserabsorbierenden Harz als Hauptkomponente ein oder mehrere Nebenkomponenten aufweist, ausgewählt aus der Gruppe bestehend aus Komplexbildnern, Deodorant, mehrwertigem Metallsalz und anorganischem Feinpartikel.

**10.** Partikelförmiges wasserabsorbierendes Mittel nach Anspruch 9, wobei der Komplexbildner ein oder mehre Mittel umfasst, ausgewählt aus der Gruppe bestehend aus Aminocarbonsäure und Salz davon.

**11.** Partikelförmiges wasserabsorbierendes Mittel nach Anspruch 9, wobei das Deodorant ein Bestandteil auf pflanzlicher Basis ist.

**12.** Partikelförmiges wasserabsorbierendes Mittel nach Anspruch 9, wobei das mehrwertige Metallsalz ein mehrwertiges Metallsalz einer organischen Säure ist.

**13.** Partikelförmiges wasserabsorbierendes Mittel nach Anspruch 9, wobei das anorganische Feinpartikel ein hydratisiertes Kompositoxid ist.

**14.** Partikelförmiges wasserabsorbierendes Mittel nach einem der Ansprüche 1 bis 13, wobei die Form der Partikel des wasserabsorbierenden Harzes eine unregelmäßig pulverisierte Form ist.

**15.** Partikelförmiges wasserabsorbierendes Mittel nach einem der Ansprüche 1 bis 14, wobei die obige (d) Zunahme der Extrahierbaren durch Alterung bis 12 Gew.% beträgt.

**16.** Partikelförmiges wasserabsorbierendes Mittel nach einem der Ansprüche 2 bis 15, wobei die obige (e) Zunahme des Verhältnisses an Extrahierbaren durch Alterung 1 bis 3 beträgt.

**17.** Ein absorbierender Artikel für Exkremente, Urin oder Blut, geformt unter Verwendung eines partikelförmigen wasserabsorbierenden Mittels nach einem der Ansprüche 1 bis 16 und hydrophilen Fasern.

**18.** Ein absorbierender Artikel für Exkremente, Urin oder Blut nach Anspruch 17, wobei der Gehalt des wasserabsorbierenden Mittels im absorbierenden Artikel 20 bis 100 % ist, bezogen auf das Gesamtgewicht des wasserabsorbierenden Mittels und hydrophiler Fasern.

**19.** Verfahren zur Herstellung eines partikelförmigen wasserabsorbierenden Mittels nach einem der Ansprüche 1 bis 16, welches umfasst
einen Schritt der vernetzenden Polymerisation der wässrigen Lösung eines ungesättigten Monomers enthaltend nicht-neutralisierte Acrylsäure und/oder Salze davon in Gegenwart eines Vernetzungsmittels und eines Kettenübertragungsmittels in einer Menge von 0,001 % bis 1 Mol-% bezogen auf die gesamten Monomere;
einen Schritt des Einstellens der Partikelgrößenverteilung; und
einen Schritt des Oberflächenvernetzens des durch die Polymerisation erhaltenen wasserabsorbierenden Harzpartikels, und welches (a) bis (c), wie unten beschrieben, genügt,

    (a) Zentrifugenretensionskapazität (CRC) der Harzpartikel in physiologischer Salzlösung im Bereich von nicht kleiner als 32 g/g;
    (b) Massenmedian der Partikelgröße (D50) des Harzpartikels im Bereich von 200 bis 400 $\mu$m; und
    (c) Anteil von Partikeln mit einem Durchmesser der Harzpartikel von kleiner als 150 $\mu$m im Bereich von 0 bis 2 Gew.-%.

**20.** Verfahren zur Herstellung eines partikelförmigen wasserabsorbierenden Mittels nach einem der Ansprüche 1 bis 16, welches umfasst
einen Schritt der vernetzenden Polymerisation der wässrigen Lösung eines ungesättigten Monomers, welches in einer Konzentration von 10 bis 30 Gew.-% nicht-neutralisierte Acrylsäure enthält in Gegenwart eines Vernetzungsmittels;
einen Schritt der Neutralisation nach der Polymerisation; und
einen Schritt der Oberflächenvernetzung des wasserabsorbierenden Harzpartikels, erhalten durch die Neutralisation und genügend (a) bis (c) wie unten beschrieben,

    (a) Zentrifugenretensionskapazität (CRC) der Harzpartikel in einer physiologischen Salzlösung im Bereich von nicht kleiner als 32 g/g;
    (b) Massenmedian der Partikelgröße (D50) des Harzpartikels im Bereich von 200 bis 400 $\mu$m; und
    (c) Anteil von Partikeln mit einem Durchmesser der Harzpartikel von kleiner als 150 $\mu$m im Bereich von 0 bis 2 Gew.-%.

21. Verfahren zur Herstellung eines partikelförmigen wasserabsorbierenden Mittels nach einem der Ansprüche 1 bis 16, aufweisend

einen Schritt der Vernetzungspolymerisation der wässrigen Lösung eines ungesättigten Monomers enthaltend nicht-neutralisierte Acrylsäure und/oder Salze davon in Gegenwart eines Vernetzungsmittels;
einen Schritt der Oberflächenvernetzung des durch die Polymerisation erhaltenen wasserabsorbierenden Harzpartikels, erhalten und genügend (a) bis (c) wie unten beschrieben,

(a) Zentrifugenretensionskapazität (CRC) der Harzpartikel in einer physiologischen Salzlösung im Bereich von nicht kleiner als 32 g/g;
(b) Massenmedian der Partikelgröße (D50) der Harzpartikel im Bereich von 200 bis 400 $\mu$m;
(c) Anteil der Partikel mit einem Durchmesser der Harzpartikel von kleiner als 150 $\mu$m ist im Bereich von 0 bis 2 Gew.-%; und

einen Schritt der Hinzufügung eines Komplexbildners an einem oder mehreren Zeitpunkten, ausgewählt aus der Gruppe bestehend aus

(i) während der Polymerisation
(ii) nach der Polymerisation und vor der Oberflächenvernetzung
(iii) während der Oberflächenvernetzung
(iv) nach der Oberflächenvernetzung.

22. Verfahren zur Herstellung nach einem der Ansprüche 1 bis 21, wobei der Schritt der Oberflächenvernetzung unter Verwendung von mehrwertigem Alkohol als Oberflächenvernetzungsmittel durchgeführt wird.

23. Verfahren zur Herstellung eines partikelförmigen wasserabsorbierenden Mittels nach einem der Ansprüche 19 bis 22, welches weiterhin den Schritt umfasst, dass man grobe Partikel mit einem Durchmesser von 400 $\mu$m bis 5000 $\mu$m und feine Partikel mit einem Durchmesser von kleiner als 150 $\mu$m aus dem wasserabsorbierenden Harz vor dem Oberflächenvernetzungsschritt entfernt.


**Revendications**

1. Agent particulaire d'absorption d'eau comprenant une résine d'absorption d'eau obtenue par polymérisation par réticulation d'un monomère insaturé contenant un groupe acide et/ou un sel de celui-ci, dans lequel ladite résine d'absorption d'eau est en outre réticulée en surface, et dans lequel ledit agent d'absorption d'eau satisfait les conditions (a) à (d) et (1) suivantes :

(a) la capacité de rétention centrifuge (CRC) de l'agent d'absorption d'eau dans une solution saline physiologique n'étant pas inférieure à 32 g/g ;
(b) la taille médiane des particules en masse (D50) de l'agent d'absorption d'eau étant dans la plage de 200 à 400 $\mu$m ;
(c) le rapport des particules de l'agent d'absorption d'eau ayant un diamètre inférieur à 150 $\mu$m étant dans la plage de 0 à 2 % en poids ;
(d) une quantité accrue des substances extractibles de l'agent d'absorption d'eau par détérioration exprimée par la formule (I) suivante de 0 à 15 % en poids et des substances extractibles de l'agent d'absorption d'eau pendant une heure dans un liquide de test de détérioration de 0,1 à 30 % en poids, dans lequel le liquide de test de détérioration désigne une solution saline physiologique contenant 0,05 % en poids d'acide L-ascorbique ;

formule (I) :

Quantité accrue de substances extractibles par détérioration (% en poids)

= substances extractibles pendant une heure dans un liquide de test de détérioration (% en poids)

- substances extractibles pendant une heure dans une solution saline physiologique (% en poids) ; et

(l) l'écart-type logarithmique de la distribution granulométrique étant dans la plage de 0,20 à 0,40.

2. Agent particulaire d'absorption d'eau selon la revendication 1, lequel agent d'absorption d'eau satisfait en outre (e) :

(e) un rapport accru des substances extractibles de l'agent d'absorption d'eau par détérioration exprimé par la formule suivante (II) de 1 à 4 fois, et des substances extractibles de l'agent d'absorption d'eau pendant une heure dans un liquide de test de détérioration de 0,1 à 30 % en poids, dans lequel le liquide de test de détérioration désigne une solution saline physiologique contenant 0,05 % en poids d'acide L-ascorbique ;

formule (II) :

Rapport accru des substances extractibles par détérioration = substances extractibles pendant une heure dans un liquide de test de détérioration (% en poids)/substances extractibles pendant une heure dans une solution saline physiologique (% en poids).

3. Agent particulaire d'absorption d'eau selon les revendications 1 ou 2, lequel agent d'absorption d'eau satisfait en outre (f) et (g) :

(f) les substances extractibles de l'agent d'absorption d'eau pendant 16 heures dans une solution saline physiologique étant dans la plage de 0,1 à 10 % en poids ;
(g) l'absorbance contre la pression à 4,8 kPa (AAP4,8 kPa) de l'agent d'absorption dans une solution saline physiologique n'étant pas inférieure à 21 g/g, de préférence n'étant pas inférieure à 22 g/g.

4. Agent particulaire d'absorption d'eau selon l'une quelconque des revendications 1 à 3, dans lequel une résine d'absorption d'eau est réticulée en surface à l'aide d'alcool polyvalent en tant qu'agent de réticulation superficielle.

5. Agent particulaire d'absorption d'eau selon l'une quelconque des revendications 1 à 4, dans lequel par ailleurs (h) de 90 à 100 % en poids d'un ageant particulaire d'absorption d'eau ont un diamètre dans la plage de 600 à 150 $\mu$m.

6. Agent particulaire d'absorption d'eau selon l'une quelconque des revendications 1 à 5, dans lequel l'agent d'absorption d'eau satisfait en outre (i) l'absorbance contre la pression à 1,9 kPa (AAP1,9 kPa) dans une solution saline physiologique n'étant pas inférieure à 25 g/g.

7. Agent particulaire d'absorption d'eau selon l'une quelconque des revendications 1 à 6, dans lequel l'agent d'absorption d'eau satisfait en outre (j) la vitesse d'absorption par procédé au vortex n'étant pas plus longue que 60 secondes.

8. Agent particulaire d'absorption d'eau selon l'une quelconque des revendications 1 à 7, dans lequel l'agent d'absorption d'eau satisfait en outre (k) la fluidité après absorption de l'humidité étant dans la plage de 0 à 20 % en poids.

9. Agent particulaire d'absorption d'eau selon l'une quelconque des revendications 1 à 8, qui comprend par ailleurs, outre une résine d'absorption d'eau en tant que composant principal, un ou plusieurs composants mineurs choisis dans le groupe constitué d'un agent chélatant, d'un désodorisant, d'un sel de métal polyvalent et d'une fine particule inorganique.

10. Agent particulaire d'absorption d'eau selon la revendication 9, dans lequel l'agent chélatant est un ou plusieurs agents choisis dans le groupe constitué de l'acide aminocarboxylique et d'un sel de celui-ci.

11. Agent particulaire d'absorption d'eau selon la revendication 9, dans lequel le désodorisant est un composant d'origine végétale.

**12.** Agent particulaire d'absorption d'eau selon la revendication 9, dans lequel le sel de métal polyvalent est un sel de métal polyvalent d'un acide organique.

**13.** Agent particulaire d'absorption d'eau selon la revendication 9, dans lequel la fine particule inorganique est un oxyde hydraté composite.

**14.** Agent particulaire d'absorption d'eau selon l'une quelconque des revendications 1 à 13, dans lequel la forme des particules d'une résine d'absorption d'eau est une forme irrégulièrement pulvérisée.

**15.** Agent particulaire d'absorption d'eau selon l'une quelconque des revendications 1 à 14, dans lequel la quantité accrue de substances extractibles par détérioration selon la condition (d) ci-dessus est de 0 à 12 % en poids.

**16.** Agent particulaire d'absorption d'eau selon l'une quelconque des revendications 2 à 15, dans lequel le rapport accru des substances extractibles par détérioration selon la condition (e) ci-dessus est de 1 à 3.

**17.** Article absorbant pour les excréments, l'urine ou le sang, qui est moulé en utilisant un agent particulaire d'absorption d'eau selon l'une quelconque des revendications 1 à 16 et des fibres hydrophiles.

**18.** Article absorbant pour les excréments, l'urine ou le sang selon la revendication 17, dans lequel une teneur de l'agent d'absorption d'eau dans l'article absorbant, sur la base du poids total de l'agent d'absorption d'eau et des fibres hydrophiles est de 20 à 100 %.

**19.** Procédé de production d'un agent particulaire d'absorption d'eau selon l'une quelconque des revendications 1 à 16, qui comprend
une étape de polymérisation par réticulation de la solution aqueuse d'un monomère insaturé contenant de l'acide acrylique non neutralisé et/ou des sels de celui-ci en présence d'un agent de réticulation et d'un agent de transfert de chaîne en une quantité de 0,001 % à 1 % en mole sur la base des monomères totaux ;
une étape d'ajustement de la distribution granulométrique ; et
une étape de réticulation superficielle de la particule de résine d'absorption d'eau obtenue par la polymérisation et satisfaisant les conditions (a) à (c) décrites ci-dessous,

(a) la capacité de rétention centrifuge (CRC) de la particule de résine dans une solution saline physiologique étant dans la plage non inférieure à 32 g/g ;
(b) la taille médiane des particules en masse (D50) de la particule de résine étant dans la plage de 200 à 400 $\mu$m ; et
(c) le rapport des particules ayant un diamètre de la particule de résine inférieur à 150 $\mu$m étant dans la plage de 0 à 2 % en poids.

**20.** Procédé de production d'un agent particulaire d'absorption d'eau selon l'une quelconque des revendications 1 à 16, qui comprend
une étape de polymérisation par réticulation de la solution aqueuse d'un monomère insaturé dans une concentration de 10 à 30 % en poids contenant de l'acide acrylique non neutralisé en présence d'un agent de réticulation ;
une étape de neutralisation après la polymérisation ; et
une étape de réticulation superficielle de la particule de résine d'absorption d'eau obtenue par la neutralisation et satisfaisant les conditions (a) à (c) décrites ci-dessous,

(a) la capacité de rétention centrifuge (CRC) de la particule de résine dans une solution saline physiologique étant dans la plage non inférieure à 32 g/g ;
(b) la taille médiane des particules en masse (D50) de la particule de résine étant dans la plage de 200 à 400 $\mu$m ; et
(c) le rapport des particules ayant un diamètre de la particule de résine inférieur à 150 $\mu$m étant dans la plage de 0 à 2 % en poids.

**21.** Procédé de production d'un agent particulaire d'absorption d'eau selon l'une quelconque des revendications 1 à 16, qui comprend
une étape de polymérisation par réticulation de la solution aqueuse d'un monomère insaturé contenant de l'acide acrylique non neutralisé et/ou des sels de celui-ci en présence d'un agent de réticulation ;
une étape de réticulation superficielle de la particule de résine d'absorption d'eau obtenue par la polymérisation et satisfaisant les conditions (a) à (c) décrites ci-dessous,

(a) la capacité de rétention centrifuge (CRC) de la particule de résine dans une solution saline physiologique étant dans la plage non inférieure à 32 g/g ;
(b) la taille médiane des particules en masse (D50) de la particule de résine étant dans la plage de 200 à 400 $\mu$m ;
(c) le rapport des particules ayant un diamètre de la particule de résine inférieur à 150 $\mu$m étant dans la plage de 0 à 2 % en poids ; et

une étape d'addition d'un agent chélatant à un ou plusieurs moments choisis dans le groupe constitué de

(i) au cours de la polymérisation
(ii) après la polymérisation et avant la réticulation superficielle
(iii) au cours de la réticulation superficielle
(iv) après la réticulation superficielle.

22. Procédé de production selon l'une quelconque des revendications 19 à 21,
dans lequel l'étape de réticulation superficielle est réalisée à l'aide d'alcool polyvalent en tant qu'agent de réticulation superficielle.

23. Procédé de production d'un agent particulaire d'absorption d'eau selon l'une quelconque des revendications 19 à 22, qui comprend par ailleurs une étape d'élimination des particules grossières ayant un diamètre de 400 $\mu$m à 5 000 $\mu$m et des particules fines ayant un diamètre inférieur à 150 $\mu$m à partir de la particule de résine d'absorption d'eau avant l'étape de réticulation superficielle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 32649 A
- GB 2267094 B
- US 5051259 A
- US 5419956 A
- US 6087002 A
- EP 0629441 A
- EP 0707603 A
- EP 0712659 A
- EP 1029886 A
- US 5462972 A
- US 5453323 A
- US 5797893 A
- US 6127454 A
- US 6184433 B
- US 6297335 B
- US 37021 A
- US 5140076 A
- US 6414214 B1
- US 5994440 A
- US 6444744 B
- US 6194531 B
- EP 0940148 A
- EP 1153656 A
- EP 0605215 A
- US 5147343 A
- US 5149335 A
- EP 0532002 A
- US 5601452 A
- US 5562646 A
- US 5669894 A
- US 6150582 A
- WO 02053198 A
- EP 0937739 A
- US 6599989 A1
- US 4093776 A
- US 4367323 A
- US 4446261 A
- US 4683274 A
- US 5244735 A
- US 4625001 A
- US 4873299 A
- US 4286082 A
- US 4973632 A
- US 4985518 A
- US 5124416 A
- US 5250640 A
- US 5264495 A
- US 5145906 A
- US 5380808 A
- EP 0811636 A
- EP 0955086 A
- EP 0922717 A
- US 6187872 B
- US 6228930 B
- US 4950692 A
- US 5478879 A
- EP 844270 A
- US 6071976 A
- US 6254990 B
- WO 2004 A
- JP 1355 A
- JP 8337726 A

### Non-patent literature cited in the description

- **JIS K.** *Testing method for water absorption speed of super absorbent resins - Description,* 1996, 7224